# EUROPEAN PATENT APPLICATION

(11) **EP 4 016 544 A1**
(43) Date of publication of application: **22.06.2022**
(21) Application number: 21210319.6
(22) Date of filing: 24.11.2021
(51) Int. Cl.: G16H 50/80, G16H 40/20, G06F 3/0482

(54) **CREDO LOGGING SYSTEM**

(30) Priority: 21.12.2020 US 202017129372
(71) Applicant: Edward Via College of Osteopathic Medicine, Blacksburg VA 24060 (US)
(72) Inventor: Garner, Harold, Virginia, 24060 (US); Rawlins, Fred, Virginia, 24141 (US)
(74) Representative: Murgitroyd & Company

(57) **Abstract**

A system implemented on a distributed computer network for capturing experiences of healthcare trainees. The system includes input devices having an interface for interacting with a user including an input screen having data input fields, selection fields and activation buttons and output screens. Also included are one or more back-end databases configured to store input data from the input devices and to provide output data to the input devices.

## Description

### BACKGROUND OF THE INVENTION

There have been many attempts, some successful and therefore sustained, to develop applications to enhance the practice of medicine for clinicians and their patients. Systems developed for procedural logging have also been developed, including, for example, automated trauma patient coding by physicians dating back to the 1990s. The systems have focused on narrow, specialized sets of codes and thus lacked wide applicability and universality. Some applications, apps, have been developed specifically to enhance medical training and capture the actions of trainees. These apps have focused on accessing a variety of medical reference materials, but have not been used for logging student activities. None of the above- mentioned systems employed universal codes to measure medical trainee clinical performance.

Current medical school curricula do not include or only touch upon billing, diagnostic or the procedural codes used in the clinic, so students, even in their clinical rotation years, are not effectively trained in the use of the codes that will be a significant part of their professional career as they begin their practice. Also, the exact breadth and depth of medical student training in their clinical rotation years is not quantified; often their experiences are captured in their loosely organized handwritten notes.

### BRIEF SUMMARY OF THE INVENTION

In one embodiment, the present invention addresses the above issues and others by providing a simple, easy to use "app" that, among other things, captures the diagnostic and procedural experiences of medical students via the internationally accepted ICD (International Statistical Classification of Diseases and Related Health Problems) system created and maintained by World Health Organization (WHO).

In other embodiments, the present invention provides an "app" to capture the clinical experiences of medical trainees and then use that information to improve clinical training of medical students, provide students with real-time feedback that they can use to enhance the depth and completeness of their training, and can be used by medical school administrators to quantify and document new accreditation requirements, identify students that are exceptional or at risk, and identify ways to improve and couple medical school learning, both basic and clinical that spans all years of medical school. As a result, better training will produce better doctors, thus improve health care quality nationally.

In other embodiments, the present invention uses Health Information Technology that captures the details of each user's clinical encounters via ICD diagnostic and procedural codes entered by trainees using electronic devices such as a mobile device. The information may be used to then dynamically enhance the training provided.

In other embodiments, the present invention uses a plurality of users at a plurality of locations involving a plurality of patient encounters and training sessions.

In a preferred embodiment, the present invention uses ICD codes to provide a common universal medical vocabulary which can capture diagnoses, procedures and drugs during patient encounters, and then use those codes to associate a variety of information (learning objectives, basic science facts, medical board review questions) to enhance student performance and evaluation.

In other embodiments, the present invention uses a spectrum of entries captured at various clinical training sites to evaluate each site and preceptor as to the quality, quantity, and variety of medical situations encountered by a user such as a student and then take action to ensure uniformity, quality, and adherence to accreditation requirements.

In other embodiments, the present invention can be used to significantly improve clinical training and thus better prepare health care workers for the reality of clinical situations.

In other embodiments, the present invention enables the sampling of public health status across a broad geographic region and across many healthcare institutions thereby presenting a new paradigm for public health monitoring with many ancillary benefits to the individuals and communities in a region.

In other embodiments, the present invention creates real-time interactive components that provide assessment information, and couple training opportunities (review facts and challenge questions) directly linked to the specific clinical patient encounters to enhance performance on medical board exams.

In other embodiments, the present invention is optimized for quick, easy and accurate entries, while being robust for a scale-up of users and translation to other medical schools.

In other embodiments, the present invention may be used to quantify and measure the uniformity of the clinical experiences across clinical rotation types, locales, and individual students to meet recent accreditation requirements.

In other embodiments, the present invention uses a native application to remove the need for continuous Internet access, thus enabling use in areas without cell phone or WIFI coverage that will sync periodically.

In other embodiments, the present invention enhances student and community public health awareness by developing analysis output, driven by student entries that provide actionable general health status for the rural socioeconomically diverse communities for a particular region.

In other embodiments, the present invention improves medicine by improving clinical training experiences. This may be done by providing an effective experience acquisition tool that provides valuable highly-relevant content back to the user, preceptors/mentors, and administrators.

Other embodiments of the present invention may be used to 1) quantify the diagnostic and procedural experience for each medical student, so that it becomes part of their permanent record (medical portfolio); 2) understand the breadth, depth, and variance of clinical experiences across all rotation specialties, rotation locales (clinics, hospitals, etc.), and students; 3) provide information to refine the clinical experiences and optimize the total training experience, especially to understand how well the curriculum objectives map to actual experiences; 4) expose students to the ICD code system, so that they are prepared to use it or similar systems as part of the Electronic Medical/Health Record (EMR/EHR) systems they will be using throughout their professional life; 5) capture and monitor in real-time the public health status of the communities in the vicinity of their training locales via accumulated statistical analysis of entries captured/sampled by the students, including trend and event trigger monitoring; and 6) capture the activities of specialized events, including off-shore medical mission trips and specialty fellows.

In other embodiments, the present invention provides a system that uses a common language/vocabulary/lexicon to associate clinical or training encounters by healthcare trainees and data that define/quantifies their progress, enhances their training and optimizes their training path. Common languages that may be used are WHO ICD codes, CPT codes, billing codes.

Other embodiments may include a collection of databases that are annotated with codes to connect patient encounters, learning materials, review materials, and performance metrics. Clinical encounters may be obtained from medical patient visits in hospitals or clinics, in transport vehicles, at retirement homes, and end of life care homes. Training encounters may include simulations, standardized patients, student-to-student exchanges, clinical skills classes, and events. Healthcare trainees may include medical students, nursing students, EMT students, fire, police, and emergency personnel. Data that defines/quantifies progress can be learning objectives, various logs (procedure, diagnostic, treatment), practice and real exams/scoring. Data that enhances training can be training materials presented to the trainee that is associated with their clinical or training encounter including matched educational material, review material, practice questions/answers/references, videos, and images. Data that optimizes training path may include evaluations of longitudinal (time-dependent) and/or measurables used to adjust or redirect training where it is most needed.

In other aspects, the present invention provides a system that uses a common language/vocabulary/lexicon to associate clinical or training encounters by healthcare trainees and data that define/quantifies the training and trainees which can then be used to adjust and optimize training and student success.

In yet aspects, the present invention provides a system that uses a common language/vocabulary/lexicon to associate clinical or training encounters by healthcare trainees.

In yet other aspects, the present invention provides a system that quantifies special or time dependence of diagnoses, procedures or drugs that are indicators of public health.

In yet other aspects, the present invention provides a system that has the ability to detect changes in dependences that indicate changes in public health; has the ability to provide summaries on public health.

In yet other aspects, the present invention provides a system that has the ability to set alerts that can inform on diagnoses, procedures or drugs which can be transmitted to people interested in public health (officials, researchers).

In yet other aspects, the present invention provides a system that includes computer or mobile device that captures and presents materials to/from trainees; a user interface; has the ability to select entries by searching hierarchical menus, recent entries, or most frequent entries by training module; has the ability to present entry associated materials for training, review, or evaluation/quantification of performance; includes one or more databases; has the ability to make annotations and associations; has training modules that include school class year, rotations, and lectures; has outputs that include searchable, filterable, sortable lists, graphs, maps; has outputs that can be delivered by download to excel or other databases or files; has outputs that include lists, graphs, or maps for school administrators; has outputs for researchers, and public health officials; includes an expanding time dependent set of databases of trainee observations and events; can be used by researcher to study training, public health, performance of drugs, procedures, diagnostics, and policy; and can be used for discovery of new biomedical associations and new drugs, procedures and diagnostics, school and government policy, and training.

In other aspects, the present invention provides a system implemented on a distributed computer network for capturing the experiences of healthcare trainees. The system includes a plurality of electronic input devices having an interface for interacting with a user including an input screen having data input fields, selection fields and activation buttons and output screens. Also included are one or more back-end databases configured to store input data from the input devices and to provide output data to the input devices.

In other aspects, the present invention provides a system including a login system having user identification routines to establish user identity and user system access status. The system may also include a common language to identify and capture clinical or training encounters by healthcare trainees, said encounters included in the input data provided to the one or more back-end databases. The common language may be ICD codes, ICD code descriptions, CPT codes, CPT code descriptions or billing codes.

In other aspects of the present invention, the CD codes provide a common medical vocabulary that captures diagnoses, procedures, and drugs during patient encounters. The system may also be configured to use said captured codes to provide predetermined learning objectives, medical information or medical board review questions to a healthcare trainee.

In other aspects of the present invention, the system 1) is configured to provide real-time interactive information to a healthcare trainee associated with an inputted ICD code, 2) includes a collection of databases containing annotated ICD codes to connect patient encounters, learning materials, review materials, and performance metrics, 3) is configured to permit healthcare trainees to search ICD codes using hierarchical menus or to permit healthcare trainees to search ICD codes using keywords, 4) configured to permit healthcare trainees to search ICD codes by presenting frequently entered ICD codes, and 5) configured to permit healthcare trainees to link data entries to a selected ICD code.

In other aspects of the present invention, the system is configured to permit healthcare trainees, for each encountered patient, the ability to input: 1) the ICD code that describes their diagnosis and treatment procedure; 2) codes for WHO listed and/or FDA approved drugs; 3) the patient gender and age; 4) free-text notes; 5) the clinic/hospital and rotation type as entered by the user and whether the training environment is a hospital or ambulatory; 6) their precise location; and 7) date/time of entry.

In other aspects of the present invention, the system is configured to include a personally identifiable information detection system, said detection system prevents entry of personally identifiable information.

In other aspects of the present invention, the system the back-end database is configured to generate reports that enable the tracking and editing of data entries.

In other aspects of the present invention, the system is 1) configured to allow administrators to monitor the cumulative entries from all trainees; 2) configured to monitor trends and trigger alerts from defined thresholds; 3) configured to include displays adapted to monitor current activities being logged and to provide statistics that track and graph system-wide usage; 4) configured to continuously process and provide data on a plurality of displays; and 5) configured to provide a healthcare trainee an interactive summary page of one or more of the entries entered by the trainee, the one or more entries are editable, searchable, and have a variety of ranked column views.

In other embodiments of the present invention, entries are associated with predetermined learning objectives or entries have predetermined codes that qualify for fulfillment.

In other embodiments of the present invention, learning objectives or log entries are fulfilled by a manual entry by a trainee with a justification that is comprised of a reference, a reading, video or a lecture.

In other embodiments of the present invention, the system is 1) configured to provide preceptors, faculty or administrators the ability to evaluate a trainee via a check off and text entry table; 2) configured to provide preceptors, faculty and administrators access to trainee log entries, summaries and learning objective and log fulfillment tables to aid in the evaluation of a trainee; 3) configured to allow evaluations to be conducted via a secure one-time link to the evaluation form; 4) configured to automatically send emails to preceptors, faculty, and administrators with a link to initiate the evaluation process; 5) configured to send reminders to evaluators if they have not submitted their evaluation by a set time; 6) configured to allow an administrator to track an evaluator's completion of one or more evaluations; and 7) configured to capture evaluation information to determine if a trainee passes or fails a rotation.

In other embodiments of the present invention, individual clinical training sites or preceptor performance or uniformity can be evaluated by the quantitative and qualitative performance of their trainees via the trainee's entries.

In other embodiments of the present invention, the system is configured to compute the average and standard deviation of trainee entries to determine if one or more trainees, preceptors or sites are significantly above or below average.

In other embodiments of the present invention, review facts are presented to trainees for review following an entry of a given diagnostic, procedure or drug code.

In other embodiments of the present invention, one or more facts are annotated by one or more codes to trigger the presentation of the one or more facts to a trainee.

In other embodiments of the present invention, review questions and answers are presented to trainees for review following an entry of a given diagnostic, procedure or drug code.

In other embodiments of the present invention, one or more facts are annotated by one or more codes to trigger the presentation of the one or more facts to a trainee.

In other embodiments of the present invention, a response by a trainee to review questions is captured as either a right or wrong answer.

In other embodiments of the present invention, a wrong answer is represented at a later time.

In other embodiments of the present invention, associations between sites, preceptors, and students for each clinical rotation are imported, generated, or maintained.

In other embodiments of the present invention, the system is 1) configured to allow a trainee to record their research experiences by entering titles, abstracts, manuscripts, data or other information regarding research activities; 2) configured to allow faculty to enter their research interests and capabilities and desire to accept a student to do research so that students can search for and identify research mentors; 3) configured to allow a trainee to enter their research interests and capabilities for comparison with faculty research interests and capabilities to identify matches; 4) configured to include a personally identifiable information detection system, said detection system prevents entry of personally identifiable information by searching for first names, last names, and patterns that resemble phone numbers, social security numbers; and 5) configured to monitor trends and trigger alerts from defined thresholds, said alerts comprising email, text or call to a person or another system.

It is to be understood that both the foregoing general description and the following detailed description are exemplary and explanatory only and are not restrictive of the invention, as claimed.

### BRIEF DESCRIPTION OF THE SEVERAL VIEWS OF THE DRAWINGS

In the drawings, which are not necessarily drawn to scale, like numerals may describe substantially similar components throughout the several views. Like numerals having different letter suffixes may represent different instances of substantially similar components. The drawings generally illustrate, by way of example, but not by way of limitation, a detailed description of certain embodiments discussed in the present document.
Figure 1 illustrates a flowchart of the overall user process for an embodiment of the present invention.
Figure 2 illustrates one user logging entry interface for an embodiment of the present invention.
Figure 3A illustrates a system summary dashboard for an embodiment of the present invention.
Figure 3B illustrates a user-editable summary table (with test data) system summary dashboard for an embodiment of the present invention.
Figure 3C illustrates a student performance metrics summary table; metrics include counts of the number of entries as well as what fraction of the top 25 most encountered diagnoses, procedures, and drugs that this student has encountered, as a measure of the breadth of their clinical exposure, for an embodiment of the present invention.
Figures 4A and 4B illustrate a database schema for an embodiment of the present invention.
Figure 5 illustrates a welcome screen for an embodiment of the present invention.
Figure 6 illustrates an exemplary screen for updating a particular rotation and location for an embodiment of the present invention.
Figure 7 illustrates an exemplary screen for entering patient demographics for an embodiment of the present invention.
Figure 8 illustrates an exemplary screen for entering the primary diagnosis for an embodiment of the present invention.
Figure 9 illustrates an exemplary screen for entering procedures for an embodiment of the present invention.
Figure 10 illustrates an exemplary screen that couples the previously entered procedures and drugs with diagnosis entries to show the user's response to a patient's diagnosis for an embodiment of the present invention.
Figure 11 illustrates an exemplary screen that allows the user to review and added information from a recent patient encounter or to start on a new patient encounter for an embodiment of the present invention.
Figure 12 illustrates an exemplary screen for a dashboard page that allows a user to review and edit entries at any time for an embodiment of the present invention.
Figure 13 illustrates an exemplary screen for editing an entry for an embodiment of the present invention.
Figure 14 illustrates an exemplary welcome screen similar to the screen described in Figure 5.
Figure 15 illustrates an exemplary screen allowing for the navigation to all user functions from the system dashboard for an embodiment of the present invention.
Figure 16 illustrates an exemplary screen of the system page that provides access to time graphs of entries in statistics, time graphs of students who are active, access to real-time student entries, summaries and map for an embodiment of the present invention.
Figure 17 illustrates an exemplary student page for an embodiment of the present invention.
Figure 18 illustrates an exemplary mentor page for an embodiment of the present invention.
Figure 19 illustrates an exemplary administrator page for an embodiment of the present invention.
Figure 20 illustrates an exemplary developer page for an embodiment of the present invention.
Figure 21 shows an exemplary screen illustrating a time graph of students who are active for an embodiment of the present invention.
Figure 22 shows an exemplary screen illustrating real-time student entries, summaries, map which may refresh every 10 seconds for an embodiment of the present invention.
Figure 23 shows an exemplary screen illustrating filtering and mapping from a User Entry Page for an embodiment of the present invention.
Figure 24 shows an exemplary screen illustrating rotation performance statistics for an embodiment of the present invention.
Figure 25 shows an exemplary screen illustrating rotation statistics-raw entries for each location for an embodiment of the present invention.
Figure 26 shows an exemplary screen illustrating location statistics-raw entries for each location for an embodiment of the present invention.
Figure 27 shows an exemplary screen illustrating the utility of the present invention for issuing public health alerts for an embodiment of the present invention.
Figure 28 shows an exemplary screen illustrating review facts and board questions presented to students triggered by a patient encounter for an embodiment of the present invention.

### DETAILED DESCRIPTION OF THE INVENTION

Detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a representative basis for teaching one skilled in the art to variously employ the present invention in virtually any appropriately detailed method, structure or system. Further, the terms and phrases used herein are not intended to be limiting, but rather to provide an understandable description of the invention.

In one embodiment, the present invention provides a web-based computer and mobile application to track the progress of trainees, monitor the effectiveness of their training locations and be a means of sampling public health status. In one preferred embodiment, the present invention uses the logging of ICD Diagnostic, Procedure and Drug codes as one of the means of tracking the experience of medical students' clinical rotations.

In another preferred embodiment, the present invention provides a system and method that may be in the form of a web-based app in which medical trainees make entries via a simple and quick interface optimized for portable electronic devices such as mobile devices and personal computers. For each patient interaction, users enter ICD diagnostic, procedure, and drug codes via a hierarchical or search entry interface. Patient demographics (age range and gender, but no personal identifiers), and free-text notes may also be inputted. Users and administrators can review and edit input via a series of output interfaces. The user interface and one or more back-end databases may be provided such as by dual redundant failover Linux servers.

In a preferred implementation, students and other users were able to master the interface in a relatively short period of around ten minutes, and thereafter complete entries in less than one minute. In one working example, five hundred-forty 3rd-year students each averaged 100 entries in the first four-week clinical rotation. Data accumulated in various disparate clinical locations has demonstrated the public health surveillance utility of the application.

This data shows that PC and mobile apps can be used to collect medical trainee experience in real-time or near real-time, quickly, and efficiently. For example, in a preferred embodiment of the present invention, after collecting 75,596 entries, less than 2% of trainees needed assistance to become proficient. Medical school administrators are also using various summaries to evaluate students and compare different rotation sites.

A primary goal of the present invention is to capture the day-to-day clinical experiences through ICD codes, by providing a user interface. The interface is configured to have operability that is quick, accurate, and efficient, so that students and other users will view this as a positive, convenient tool, as opposed to a time-consuming burden.

The data captured was a balance between what is needed to meet the goals of the system, and that which can be quickly and easily entered by users, so as not to over-burden them. Key areas were identified, such as a simple, straightforward human interface, optimized code entry methods and clear output summaries that enable individual performance monitoring. Another constraint was to create a system that did not require HIPAA compliance, so the design captured enough meaningful patient demographics to understand the relevant diagnoses and procedures logged, without needing Personally Identifiable Information, PII.

The design of the user interface and back-end database and the hardware capabilities, including speed, internet bandwidth, and reliability were prime considerations. For universal applicability across various PC types and mobile devices, a browser may be used. Also, the presentation and operation may be separately optimized for large (PC) and small (mobile device) screens. Another consideration was entry compatibility and speed across all display form factors. A preferred implementation requires Internet access. However, a native application may also be used to remove the need for continuous Internet access, something not always available in remote locations.

In another version, the logging system or app may be implemented through a browser page that quickly and easily captures trainee (or physician) experiences via user input of ICD codes that have been developed, tested, and is in use. The system syncs with a SQL back-end database, allowing for the accommodation of an effectively unlimited number of users. In one embodiment, there are over 1300 users.

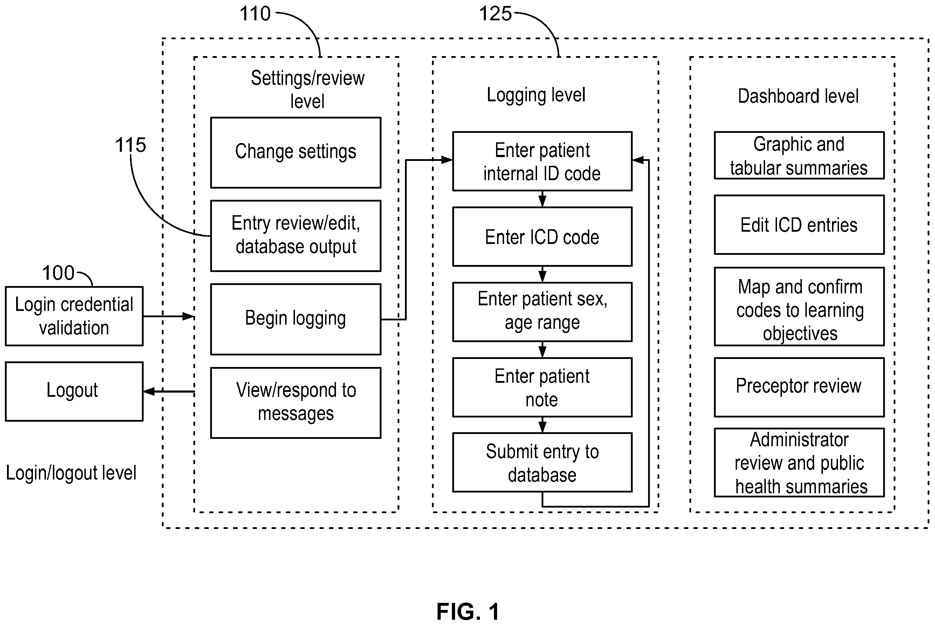

The user navigation flowchart is provided in Figure 1. There are four primary ways in which users interact with the app after login: 1) they set their rotation type and location via the settings page; 2) they create new patient ICD log entries via the logging page; 3) the can measure their quantitative performance relative to all other medical trainees for each clinical rotation; and 4) they update and associate logged data with clinical learning experiences and build an example test bank of questions via the dashboard.

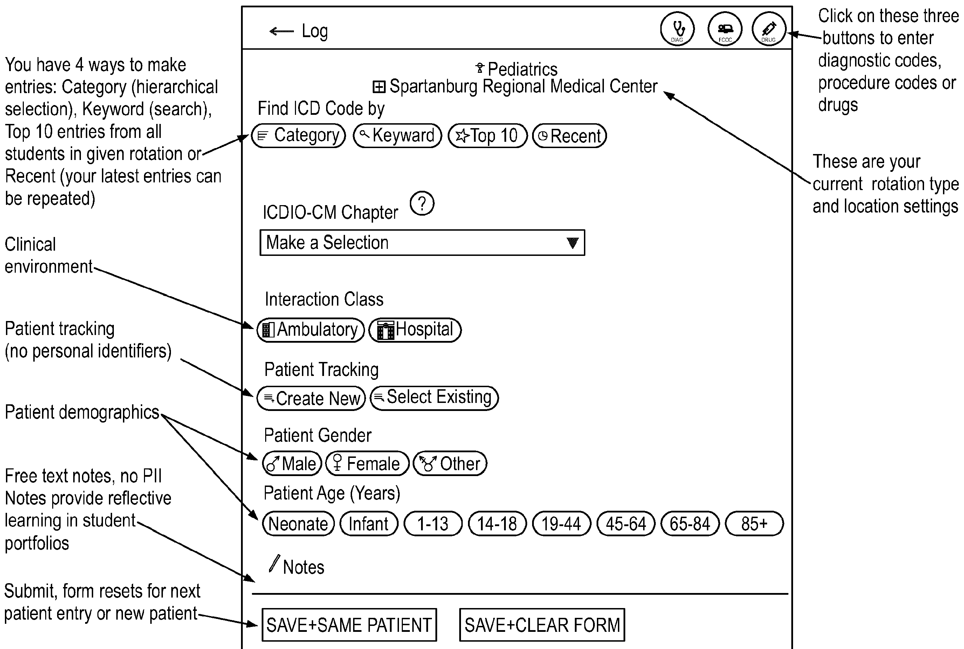

The primary web page used for logging provides the user with several methods for identifying the ICD code for a given entry - a hierarchical series of drop-down menus, a keyword-based search system; a list of the "Top 25" most frequent codes seen by all trainees in each rotation; and a recent entry selection. The system collects the following information from each user (student) as shown in Figure 2. For each patient they experience; they select/ enter the ICD code that describes their diagnosis and treatment procedure; and codes for WHO listed and/or FDA approved drugs; 2) the patient gender (M/F/O) and age (in relevant increments); 3) free-text notes; 4) the clinic/hospital and rotation type as entered by the user and whether the training environment is a hospital or ambulatory; 5) their precise location (as reported by the user's device); and 6) date/time of entry (populated using the server's time).

Users are able to select codes that would normally be considered non-billable to allow for deliberate ambiguity when a code of greater specificity is unfamiliar to the user or when there is insufficient clinical evidence for greater specificity. No personally identifiable information (PII) is collected on patients. A PII detection system prevents entry of PII into the free-text "Notes." The system does not allow entries to contain text that includes patterns of numbers typical of phone numbers and social security numbers; or proper names. To do this, a database of 85,269 proper names was constructed from the analysis of the US Census; excluding certain common words that could also be proper names (certain stop words); and excluding eponymously-named diseases; e.g., Huntington's. The intent was not to create a new Electronic Health Records (EHR) system; which would require HIPAA compliance; but could limit acceptance of such a system in our many remote sites; if it was viewed as competing with existing systems.

The back-end database may be configured to generate reports that enable users to track and edit their entries; allows administrators to monitor the cumulative entries from all students; monitor trends; and trigger alerts from defined thresholds.

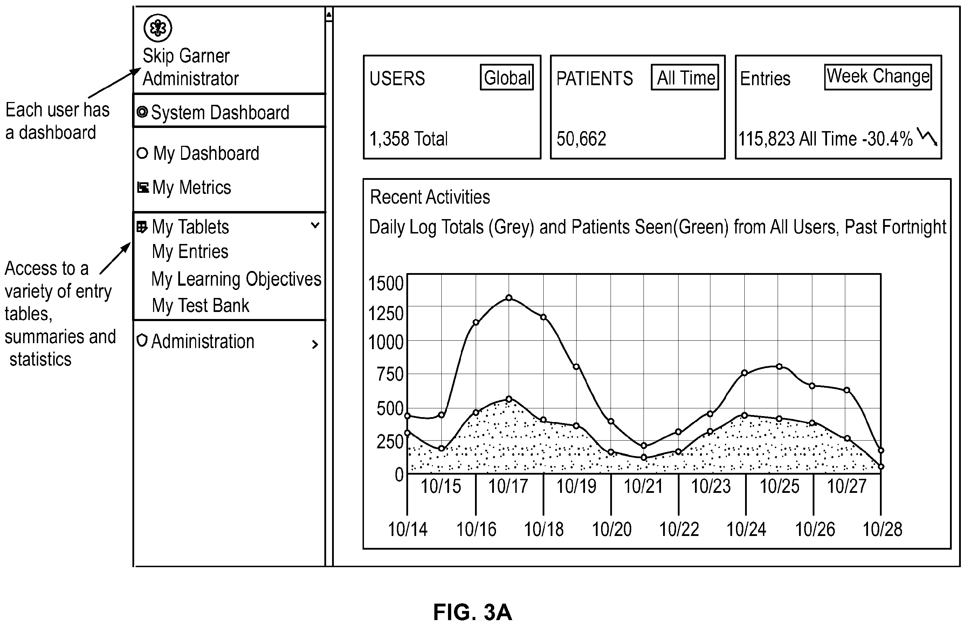

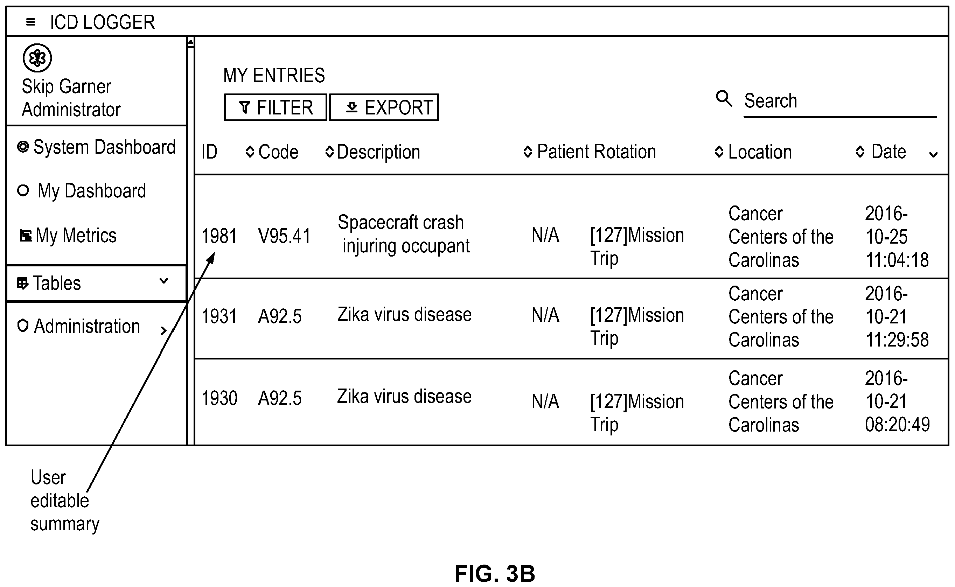

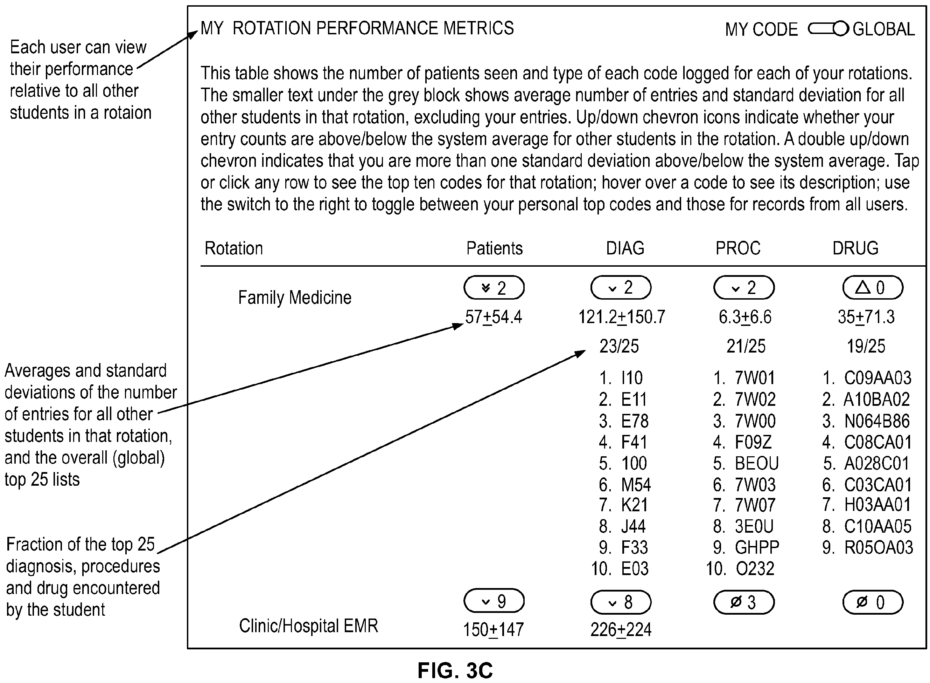

As shown in Figures 3A-3C, the system may include displays designed to monitor the current activities being logged (codes; date and time only) and statistics that track and graph system-wide usage which can be monitored by administrators or as public displays so that students and visitors at the medical school can get an instantaneous view of clinical activities.

The servers on which the system runs may be configured as two identical servers with fail-over capability, and other off-site backup and mirroring. Data interchange with the app's front-end interface is asynchronous to allow for rapid entry. The database, which may be MySQL, captures all the user logged information, and also contains all the user access information and other information required for the various displays.

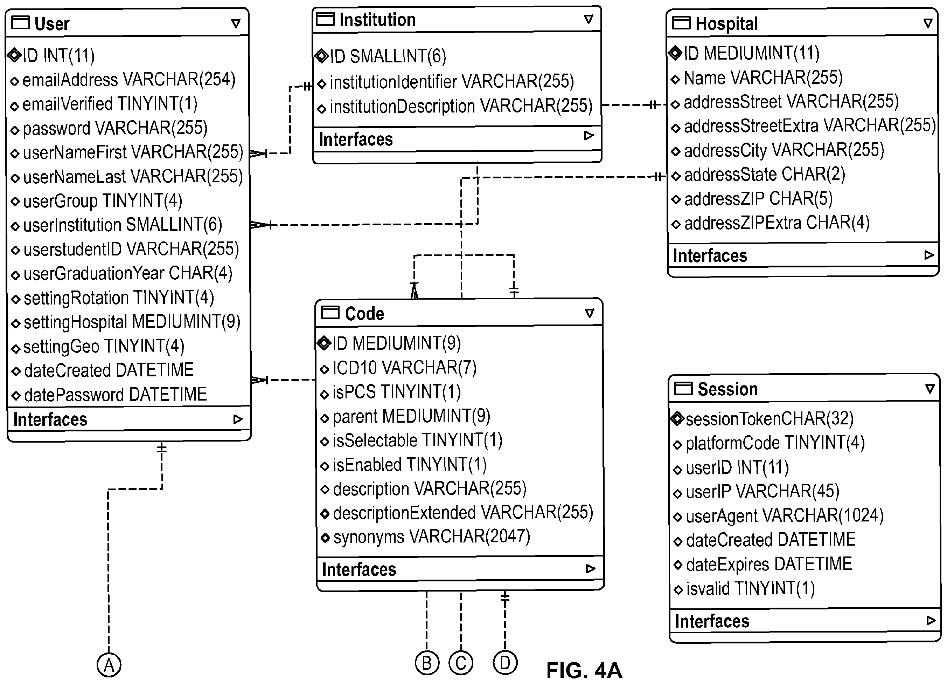

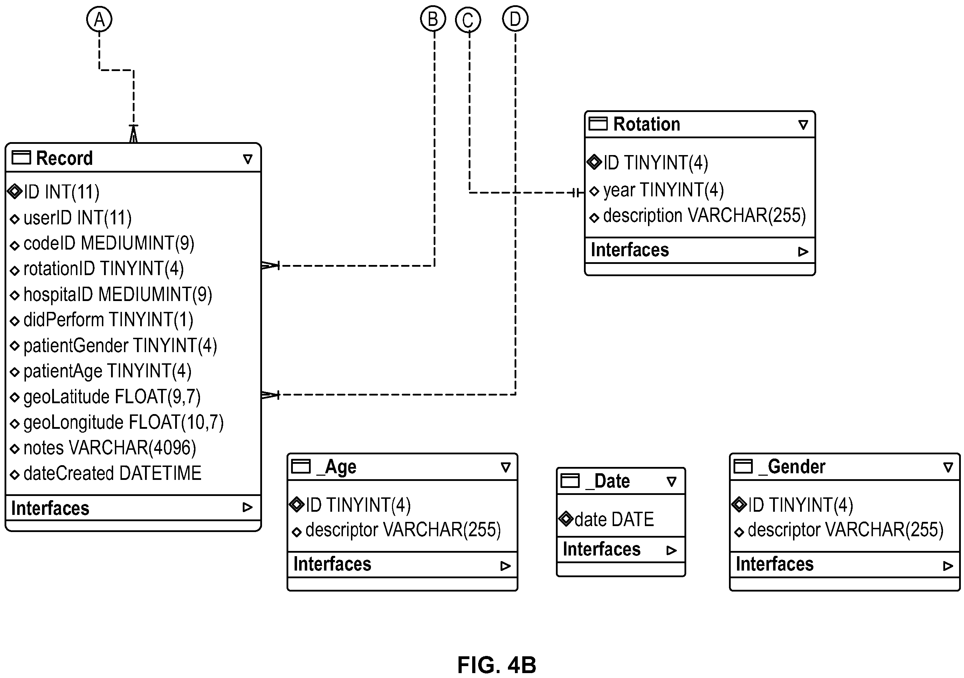

The database schema is given in Figure 4. For one embodiment, the entire database (including entries and ICD codes) maybe around 70.8 megabytes. Each new entry (including its index) on average is 0.25 kilobytes. The app uses approximately 1.2 megabytes on initial load, but with caching and mod_pagespeed optimization, subsequent loads are only 34 kilobytes. Thus, the system (and associated bandwidth to/from servers) was designed for hundreds of simultaneous queries by users, for greater than 50,000 users, following stress tests conducted using Loader.

Content that may be used with an embodiment of the system is given in Table 1, including both diagnostic and procedural codes, retrieved from the WHO website, and drugs downloaded from the WHO ATC website, and FDA approved drugs web site.

**Table 1**

| Content of the data tables. | |
|---|---|
| | |
| ICD-10 Diagnostic Codes | 44,221 |
| ICD-10 Procedure Codes | 78,705 |
| WHO/FDA Approved Drugs | 4823 |
| Student Rotation Sites | > 700 |
| Student Rotation Types | > 80 |
| Learning Objectives | > 500 |
| Student Users | > 1200 |
| Faculty/Admin Users | > 100 |

All codes are stored in the local database for maintaining referential integrity and providing detail-rich reporting functionality. For example, at the VCOM medical school, there are over 80 recognized rotation and activity types; these activities are conducted at over 700 different locations (clinics, hospitals, etc.). There are program-specific tables which house rotation types and specific clinical locations, as well as administrative tables to maintain user entities, granular access rights, forgotten password recovery codes, etc.

A keyword search procedure may be implemented through the use of the MySQL engine's native full-text index. The ICD descriptions were supplemented with synonyms to ease and speed finding the most specific code and enable common medical and non-medical terms to be used.

In another embodiment, data may be continuously processed and provided on a variety of displays (web pages) which may be in the form of one or more dashboards. Each student or user is provided with an interactive summary page of all their entries, which is editable, searchable, and has a variety of ranked column views. Students or users can also monitor their performance relative to all other students in the same rotation. Performance reports contain the number of entries made by the student or users and the fraction of the top 25 diagnoses, procedures and drugs which they have encountered as a measure of the breadth of clinical experience relative to all other students or users in a given rotation. There are also display pages for overall system use, recent entries, and other information. The full set of user entries can be exported to an Excel spreadsheet so that a variety of custom analyses can be performed by system administrators or faculty who wish to study the activities of the students during their various activities.

In actual use, it has been found that it initially takes approximately ten minutes to master and begin logging. Thereafter the time required to make new log entries drops to < 1 min/entry and users primarily select ICD codes using the search feature. Diagnostic entry selection is easier than procedure entries, especially as more specificity is required. Students interact with the primary log entry page over 95% of the time, and only rarely review and edit/update entries via the dashboard interface.

In one use that was studied, a team of eight 3rd year medical students was engaged to log their experiences and provide feedback. Students have completed two clinical rotations (family medicine, internal medicine, psychiatry, geriatrics, pediatrics, etc.) of one-month duration each at 13 different hospital/clinic sites. They have entered 2623 diagnostic, procedure and drug codes in the 40 days they have been in the clinic. The student entry rate varied significantly, between 97 and 916 entries, and it did not correlate with any particular type of rotation, but appears to reflect the variation in rigor among students. There were a total of 586 drug codes entered, with the most frequent being Amoxicillin, Cefdinir, Ondansetron and various forms of Acetaminophen. The most frequent diagnostic codes were (110) essential primary hypertension, (E78x) various forms of hyperlipidemia, (E11x) various forms of Type 2 Diabetes, and (Bx) various viral infections. This sampling of diagnoses reflects the known obesity prevalence in the area monitored.

An additional system utility demonstration was obtained from 2nd-year students that participated in medical outreach trips to El Salvador, Honduras, and the Dominican Republic, where VCOM maintains a permanent local presence at its clinics. There have been ten mission trips, 7211 codes were entered by 239 student users.

This has also provided insight into the Public Health potential for the present invention which has enabled fundamental observations: Females come to the clinics at twice the rate of males; the code (110) essential primary hypertension was the most frequent, as expected given the worldwide obesity crisis. Given the socioeconomic level of the indigenous population that visit the clinics, it was not surprising to see that the next most frequent entries included were (J069) acute upper respiratory infection, (B89) unspecified parasitic disease, and (K21) gastroesophageal reflux disease with esophagitis, for these are typically seen in first-world populations. There were many unique codes logged, including (A30) Leprosy, and many genetic or complex disorders: (Q90) Down's syndrome, (C5061) malignant breast neoplasm, and (Q66) congenital deformities of the feet, to name a few

Students and users have accepted the app as their primary method for capturing their clinical experiences and building their experience portfolio. The app, therefore also takes on a role in the formal documentation for the medical school. Preceptors may use the ICD Logger of the present invention in reviewing student performance during a given rotation, and also use this information as an opportunity to engage students in reflective learning based on cases they have logged. Administrators can also evaluate the uniformity of the clinical experiences across various training sites using this data, both quantitatively (number of entries) and considering the spectrum of different clinical cases encountered (using the top 10 list).

The embodiments of the present invention may be used in many ways, including use in other medical training areas such as Emergency Medical Technicians, nursing, and dentistry. For these other allied health professions require clinical experiences, which could be captured by using codes such as ICD-10 codes. The embodiments of the present invention may also be used in other medical/non-medical areas such as billing/coding training, survey taking, etc.

The embodiments of the present invention may also be used as a public health monitoring/surveillance tool, for the data emerging is consistent with the general population health in the areas surveyed.

In yet another use, data collection was completed every week, and a designated doctor manually uploaded the patients seen during the week from the HealthMaster database, a locally developed and established EMR system, and to the system of the present invention. The similarities and differences between the two underserved sites are shown in Table 2.

**TABLE 2: The Top 10 Diagnoses for the Ghana and Appalachia sites. Using the "Top 10" feature in the Logger page, these lists were composed to demonstrate the similarities and differences between the locations:**

| **Clinic Rotation at HMC** | **Family Medicine in Appalachia** |
|---|---|
| Top 10 Diagnoses | Top 10 Diagnoses |
| • Plasmodium falciparum malaria | • Encounter for general examination without complaint, suspected or reported diagnosis |
| • Essential (primary) hypertension | • Essential (primary) hypertension |
| • Other disorders of urinary system | • Type 2 diabetes mellitus |
| • Acute upper respiratory infections of multiple and unspecified sites | • Disorders of lipoprotein metabolism and other lipidemias |
| • Other anemias | • Biochemical lesions, not elsewhere classified |
| • Disorders of lipoprotein metabolism and other lipidemias | • Dorsalgia |
| • Vasomotor and allergic rhinitis | • Other joint disorder, not elsewhere classified |
| • Other and unspecified soft tissue disorders, not elsewhere classified | • Persons encountering health services in other circumstances |
| • Unspecified acute lower respiratory infection | • Encounter for screening of malignant neoplasms |
| • Open would of head | • Long term (current) drug therapy |

| Top 10 Prescriptions | Top 10 Prescriptions |
|---|---|
| • Ibuprofen | • Lisinopril |
| • Ciprofloxacin | • Levothryoxine sodium |
| • Cetirizine | • Amoxicillin |
| • Folic acid | • Gabapentin |
| • Metronidazole | • Dexamethasone |
| • Amoxicillin | • Atorvastatin |
| • Diazepam | • Hydrochlorothiazide |
| • Bendroflumethiazide | • Azithromycin |
| • Naproxen | • Prednisone |
| • Cefuroxime | • |

Tema, Ghana is considered a suburban locality, with the most populous area being Community 1. Appalachia was chosen because it is a socioeconomically disadvantaged population in the US; in some respects, it has similarities to a third-world country. Both sites have essential (primary) hypertension as their second most frequent diagnosis as well as disorders of lipoprotein metabolism and other lipidemias, which can be attributed to the increasing rate of obesity and lack of exercise. Similar drugs used to treat these conditions are frequently administered at both sites, such as Bendroflumethiazide, Hydrochlorothiazide, Atorvastin, and Lisinopril.

They both use various antibiotics, such as Amoxicillin, Ciprofloxacin, and Azithromycin. However, the use of these drugs reflects the differences between diagnoses at the two sites. Although it is advanced, HMC Ghana has limited resources and cannot always obtain the best medications, limiting what prescriptions are available to the patients. Some of the diagnoses made in Ghana are respiratory in nature, possibly due to the working conditions, excessive dust in the environment and the plurality of viral infections in tropical environments. The prescriptions made to the patients in Appalachia are more orthopedic; for example, the diagnoses of dorsalgia and other joint disorders. Regular visitation of the doctor's office or "check-ups" (encounter for general examination without complaint) is not common in Ghana, due to the cultural standard of if one is not visibly ill, there is no need to go to the doctor.

Most developing countries lack the technical expertise, funding and technological infrastructure needed for implementing EMR systems. However, some countries have proven that is possible to have a locally developed EMR, such as the one in Ghana. This EMR functions very well for the size of the clinic, although it does have technical issues with certain features. Using the system and methods of the present invention supports an established system by providing back-up on a server that is less likely to experience technical issues. It also provides a universal medical language (ICD codes) that enables worldwide comparisons. Due to its transparent and intuitive design and minimal cost, the implementation of the present invention would be beneficial for all concerned. The present invention may be used to provide public health data at a per-patient resolution for future research for a predetermined location such as the Sub-Saharan, and a facility or institution would have a secure back-up of their patient information centrally located outside of the country. Transcribing data from one EMR system to another is an arduous and prolonged task. However, capturing universal information (ICD codes) for a patient and having a separate backup it was concluded that bi-weekly data conversion and collection of information was considered valuable. After initial tests, we the best option.

The embodiments of the present invention may be used as an ancillary EMR system as was shown by testing in a new hospital in Ghana. This allowed the hospital to have an off-site backup of critical medical records. Capturing the information universally, using WHO's ICD-10 codes, enables the present invention to compare the public health picture in a predetermined location such as Ghana relative to a US population.

The embodiments of the present invention may also be used as a stand-alone EMR system, sufficient for developing nations, provided that it is accompanied with a database that relates the internal CREDO ICD Logger patient ID with their true ID.

By keeping the conversion database local, confidential and encrypted, it is possible to separate the bulk of the patient information via ICD-10 codes from the confidential information. This, in turn, demonstrates that a large portion of the information, information of particular value to continuously monitoring and surveying public health down to individual anonymous patients is possible.

Login/logout level pseudo-code for use at step 100 of Figure 1 may include:
- Users log in with a username and password
- After login, users are in the settings level
- After a user is finished logging or viewing reports, they can return to the settings level and then log off

Settings level 110, as shown in Figure 1, pseudocode may include:
- Administrators can set alerts, request database download
- Settings allow a user to change a password, location (including a selection from a pre-determined set of GPS coordinates obtained from a mobile device), rotation; settings are saved, exit settings and return to settings/logoff level
- User can view about and help
- A user can log off
- User can view and respond to messages
- User can view various database reports (output)
- User can go to entry-level Enter settings level
- User can go to settings, messages, about, help, reports, logoff

Database reports 115, as shown in Figure 1, pseudocode may include:
- User can enter reports level and request several types of view summaries
- User entries over time, by code, by location, by sex, by age group
- Administrators can enter reports level and request several types of view summaries
- Administrators can request entries by a student, location, code, time, location, sex, age; and various sets of these
- Administrators can view alerts

Backend database and updater pseudocode and schema Backend database contents may include:
- Table of users, passwords, and privileges
- Table of locations (hospitals, clinics, remote places, GPS coordinates)
- Table of ICD codes (number, hierarchy, description)
- Table of entries (entry ID, ICD code entered, M/F/O, age, location, user, time/date stamp, rotation name)
- Database logic
- Consistency checking
- Alert detection
- Updater (administrative control)
- Table loader of new/updated set of ICD codes, new/change/remove users, set of locations, set of rotation names)
- Ability to edit, change, add, delete codes, users, locations, rotation names
- Downloader and backup (administrative control)
- Export databases to excel
- Backup and sync database across servers

Logging level 125, as shown in Figure 1, pseudocode may include:
- Logging level entered
- User can enter with pulldown menus, buttons and text entry boxes in any order ICD code, sex, age, notes; and optionally not sex, age, notes
- Entry of ICD code can use search for a code
- Entry of ICD code can use autocomplete
- Entry of ICD code can use recent entries
- User submits with button, which makes entry into a database, and resets logging page for another entry, i.e., 2 and 3 loops until exit this routine
- Exit routine back to settings level

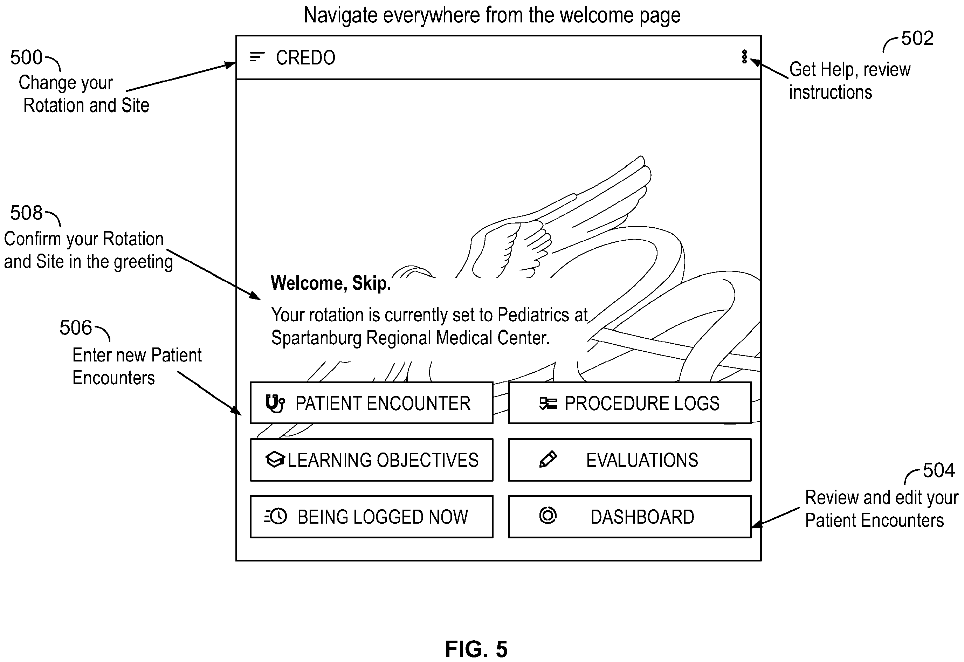

Figures 5-28 illustrate exemplary web pages or screens that may be used with various embodiments of the present invention. Figure 5 shows a welcome screen. It provides areas, sections, or buttons where a user may change rotation and location 500, a section where help and review instructions may be obtained 502, dashboard access 504 where the user can review and edit patient encounters, patient encounter access 506 where a user can enter patient-specific information discussed below, and the screen also provides the opportunity to confirm the rotation and location 508.

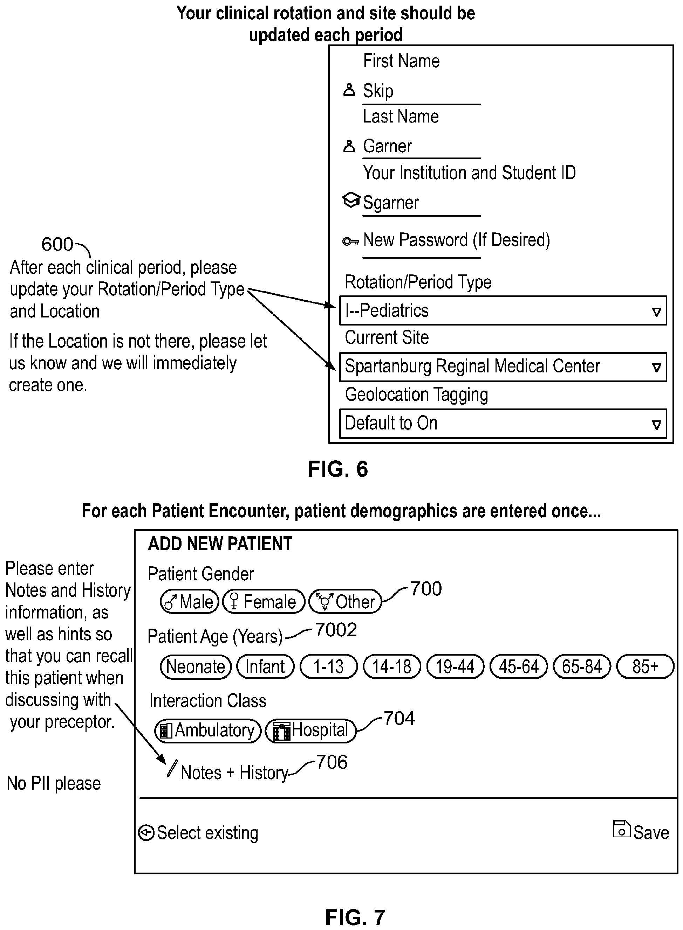

Figure 6 illustrates an exemplary screen for updating a particular rotation and location. To update the rotation and location, the account setting screen is used where a user is able to select a particular rotation type and location of the rotation 600.

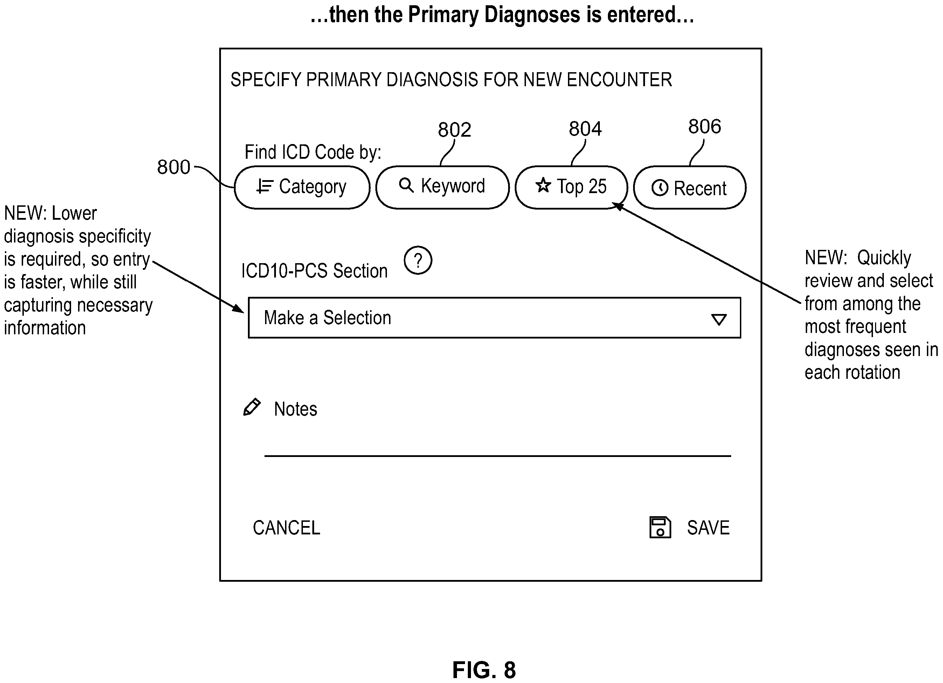

Figures 7-11 illustrate exemplary screens for entering patient encounter information where a user is led through a series of steps where diagnoses, procedures, and drugs for each patient are entered. Figure 7 illustrates a screening for entering patient demographics such as gender 700, age 702, interaction class 704 and a section for adding notes and history 706. Figure 8 illustrates entering the primary diagnosis. As shown, ICD codes may be determined by category 800, keywords 802, top 25 or most frequent diagnosis seen in the rotation 804, and most recent 806.

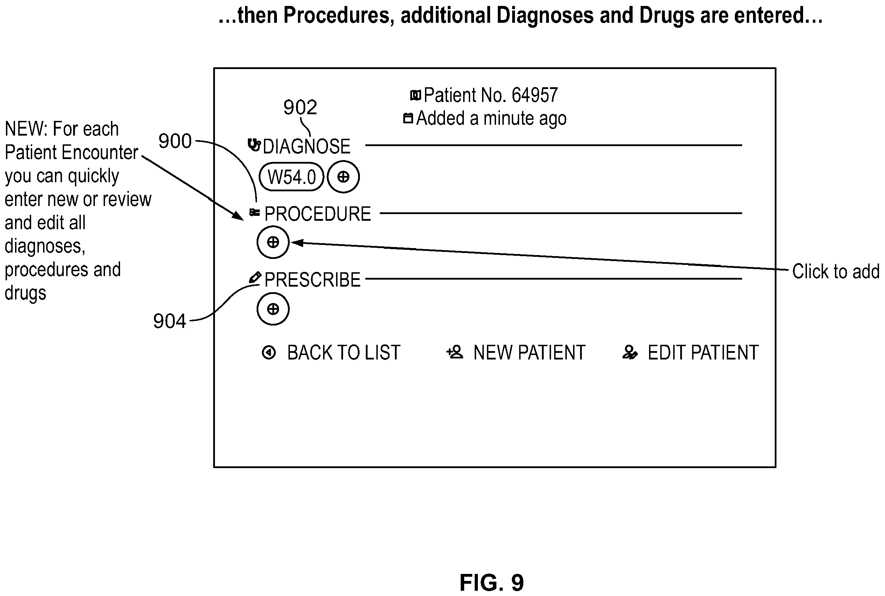

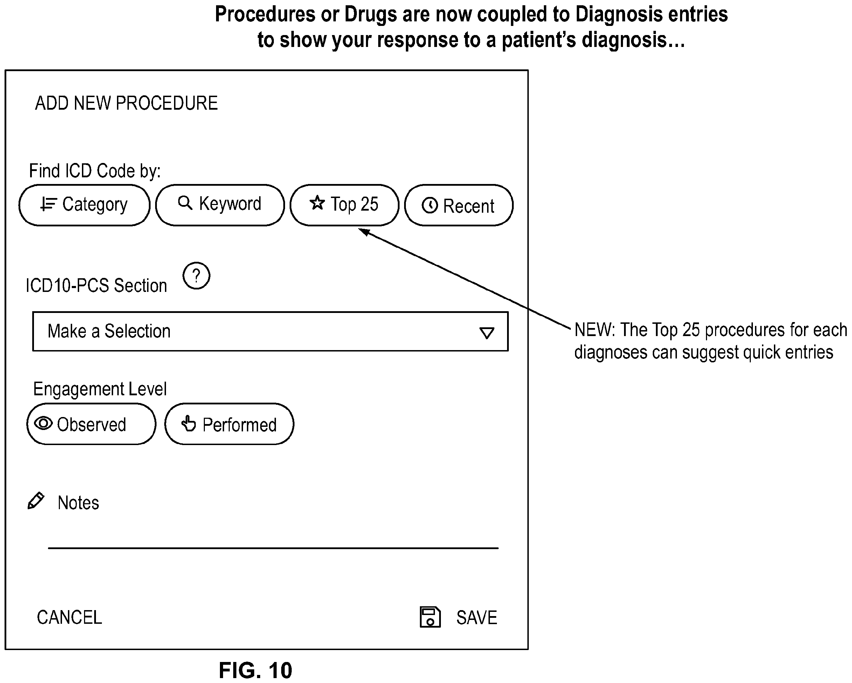

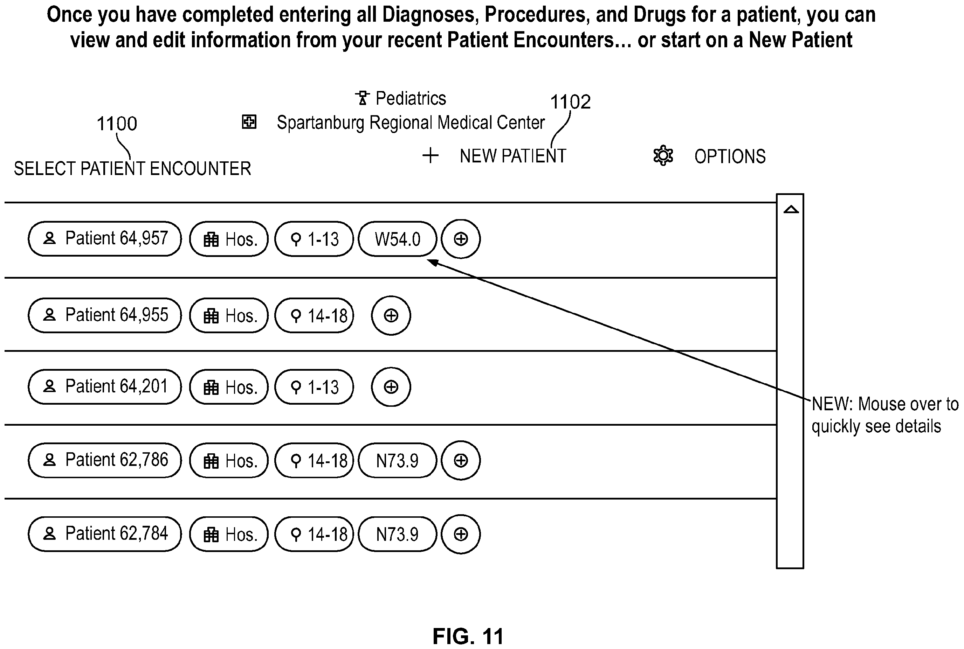

Figure 9 illustrates an exemplary screen for entering procedures 900, one or more additional diagnoses 902 and drugs 904. Figure 10 illustrates an exemplary screen that couples the previously entered procedures and drugs with diagnosis entries to show the user's response to a patient's diagnosis. Figure 11 illustrates an exemplary screen that allows the user to review and added information from recent patient encounter 1100 or to start on a new patient encounter 1102.

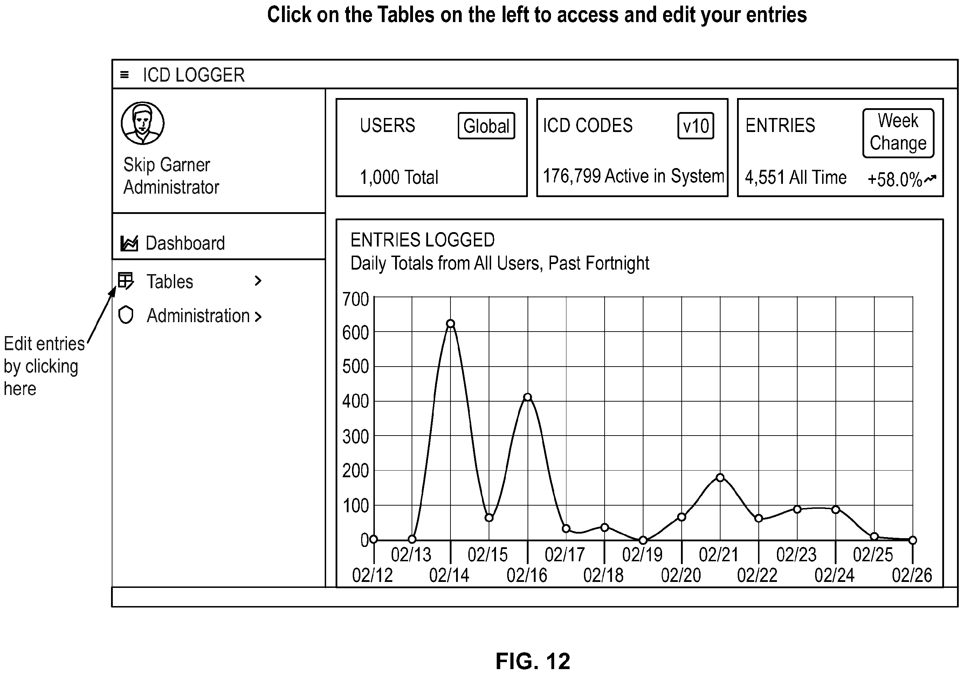

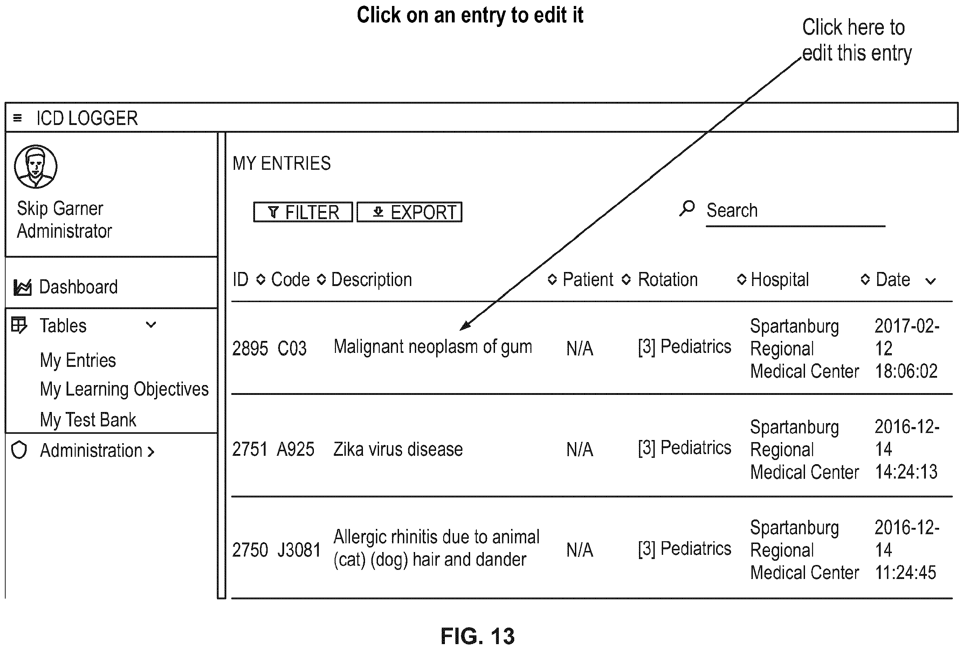

Figure 12 illustrates an exemplary screen for a dashboard page that allows a user to review and edit entries at any time. Information that may be provided on the dashboard includes the ability to edit entries and review tables and charts providing global and individual information. Figure 13 is an exemplary screen for editing an entry.

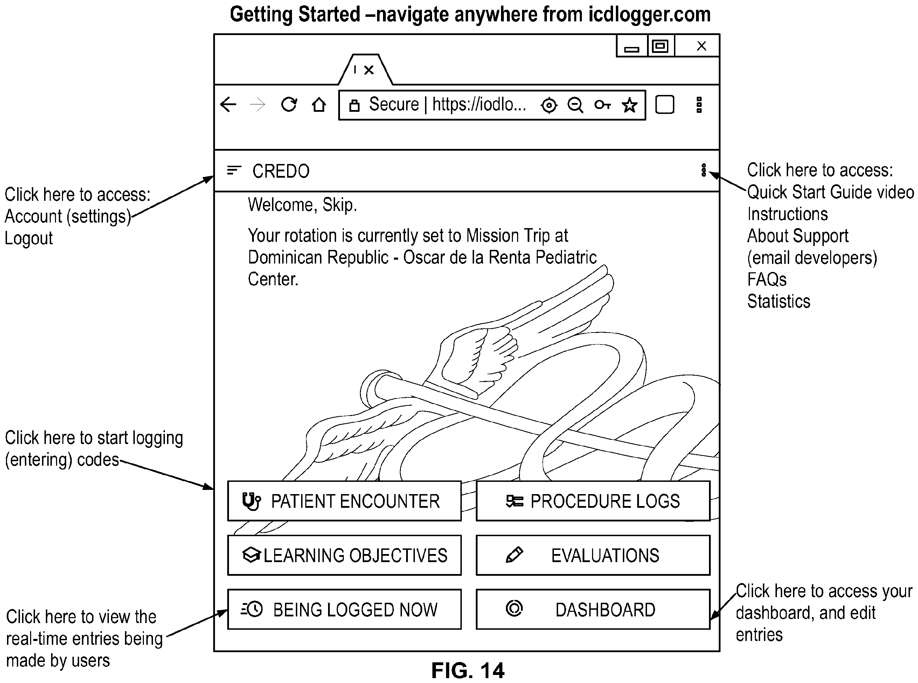

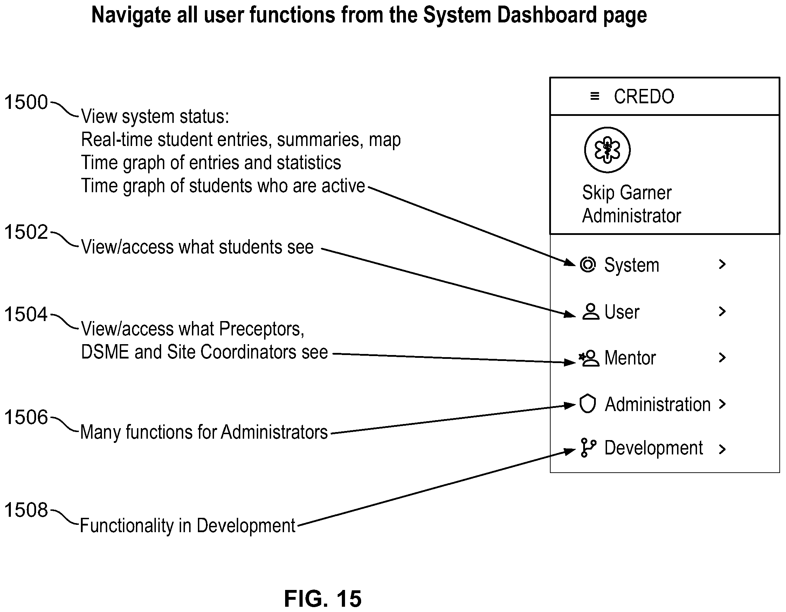

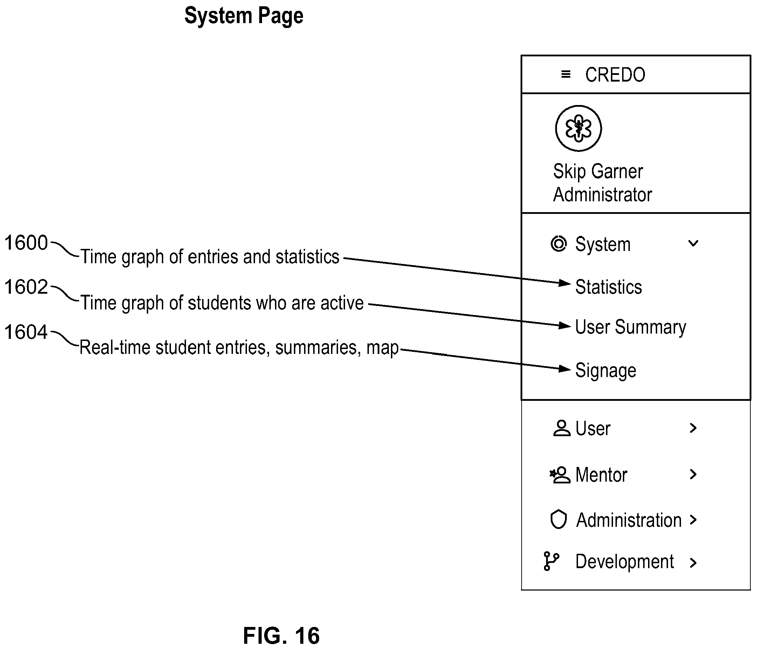

While Figures 5-13 are primarily directed towards student users, Figures 14-28 are primarily directed to administrative functions but may be used by student users as well. Figure 14 is an exemplary welcome screen similar to the screen described in Figure 5. Figure 15 illustrates an exemplary screen allowing for the navigation to all user functions from the system dashboard. System access 1500 allows access to real-time student entries, summaries, maps, time graphs of entries and statistics, and time graphs of active students. User button 1502 allows for reviewing and access to see what a particular student sees. Button 1504 allows a user to review and access what preceptors, DSME and site coordinators see. Button 1506 provides for access to administrative functions, and button 1508 is reserved for functionality development. Figure 16 is an exemplary screen of the system page that provides access to time graphs of entries in statistics 1600, time graphs of students who are active 1602, and access to real-time student entries, summaries and map 1604.

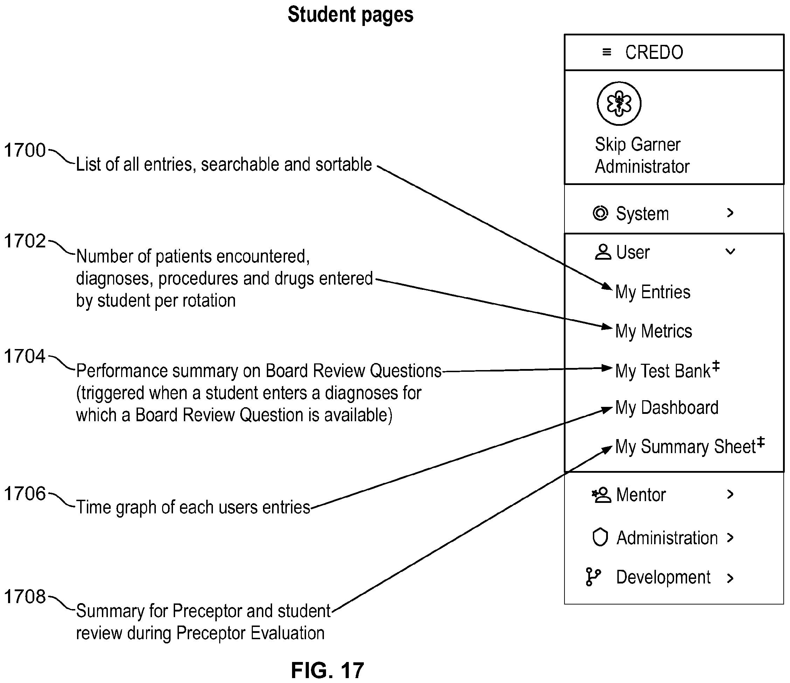

Figure 17 illustrates an exemplary student page. Buttons that may be provided include 1700 List of all entries, searchable and sortable; 1702 Number of patients encountered,
diagnoses, procedures and drugs entered by a student per rotation; 1704 Performance summary on Board Review Questions (triggered when a student enters diagnoses for which a Board Review Question is available); 1706 Time graph of each entry; 1708 Summary for Preceptor and student review during Preceptor Evaluation.

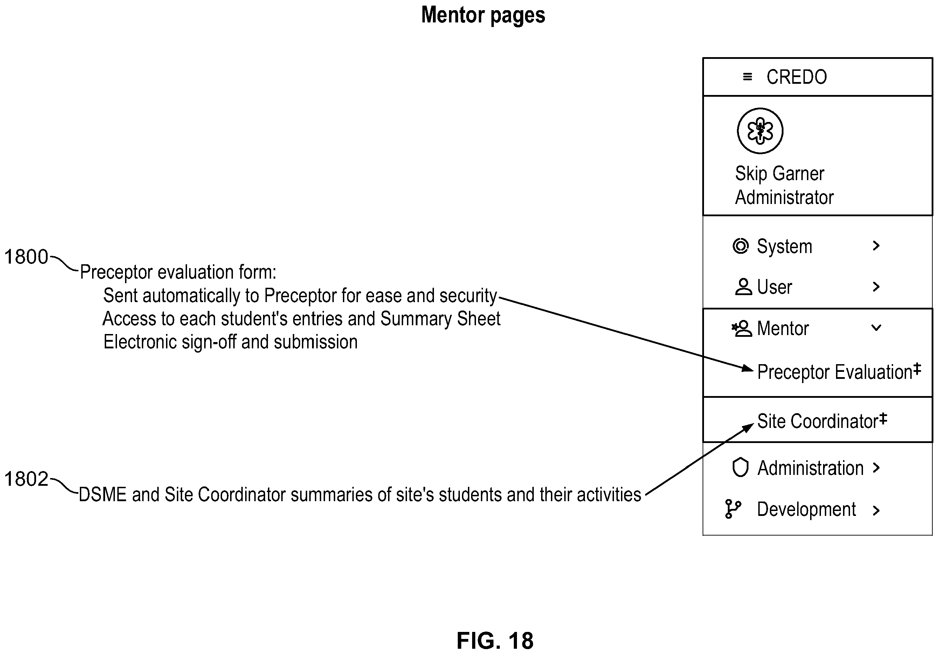

Figure 18 illustrates an exemplary mentor page. Buttons that may be provided include: 1800 Preceptor evaluation form, sent automatically to Preceptor for ease and security, access to each student's entries and summary sheet and electronic sign-off and submission; and 1802 DSME and Site Coordinator summaries of site's students and their activities.

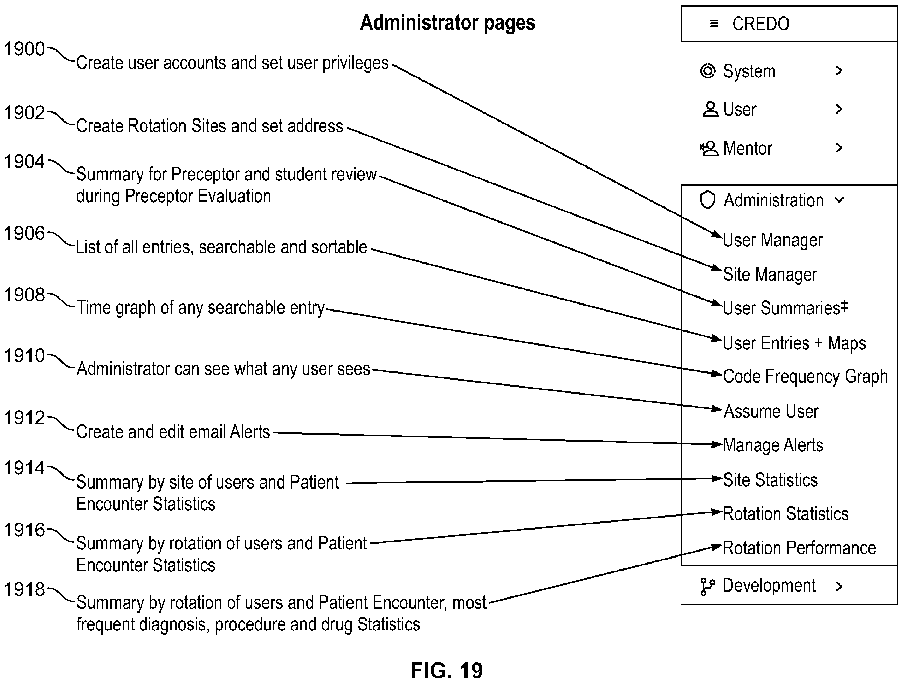

Figure 19 illustrates an exemplary administrator page. Buttons that may be provided include: 1900 Create user accounts and set user privileges; 1902 Create Rotation Sites and set address; 1904 Summary for Preceptor and student review during Preceptor Evaluation; 1906 List of all entries, searchable and sortable; 1980 Time graph of any searchable entry; 1910 Administrator can see what any user sees; 1912 Create and edit email Alerts; 1914 Summary by the site of users and Patient Encounter Statistics; 1916 Summary by rotation of users and Patient Encounter Statistics; and 1920 Summary by rotation of users and Patient Encounter, most
frequent diagnosis, procedure and drug Statistics.

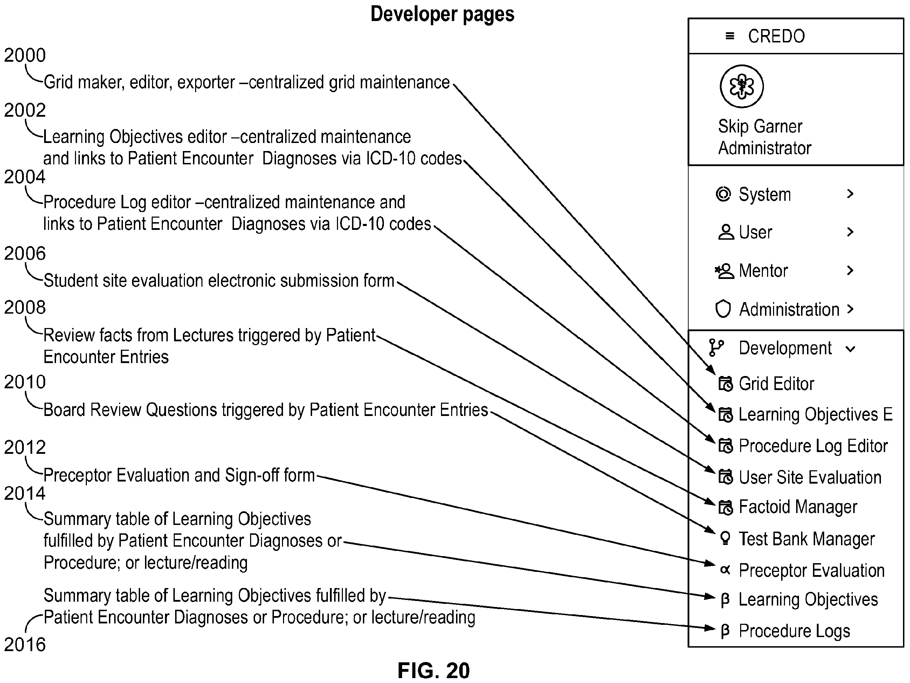

Figure 20 illustrates an exemplary developer page. Buttons that may be provided include: 2000 Grid maker, editor, exporter - centralized grid maintenance; 2002 Learning Objectives editor - centralized maintenance and links to Patient Encounter Diagnoses via ICD codes; 2004 Procedure Log editor - centralized maintenance and links to Patient Encounter Diagnoses via ICD codes; 2006 Student site evaluation electronic submission form; 2008 Review facts from Lectures triggered by PatientEncounter Entries; 2010 Board Review Questions triggered by Patient Encounter Entries; 2012 Preceptor Evaluation and Sign-off form; 2014 Summary table of Learning Objectives fulfilled by Patient Encounter Diagnoses or Procedure; or lecture/reading; and 2016 Summary table of Learning Objectives fulfilled by Patient Encounter Diagnoses or Procedure; or lecture/reading.

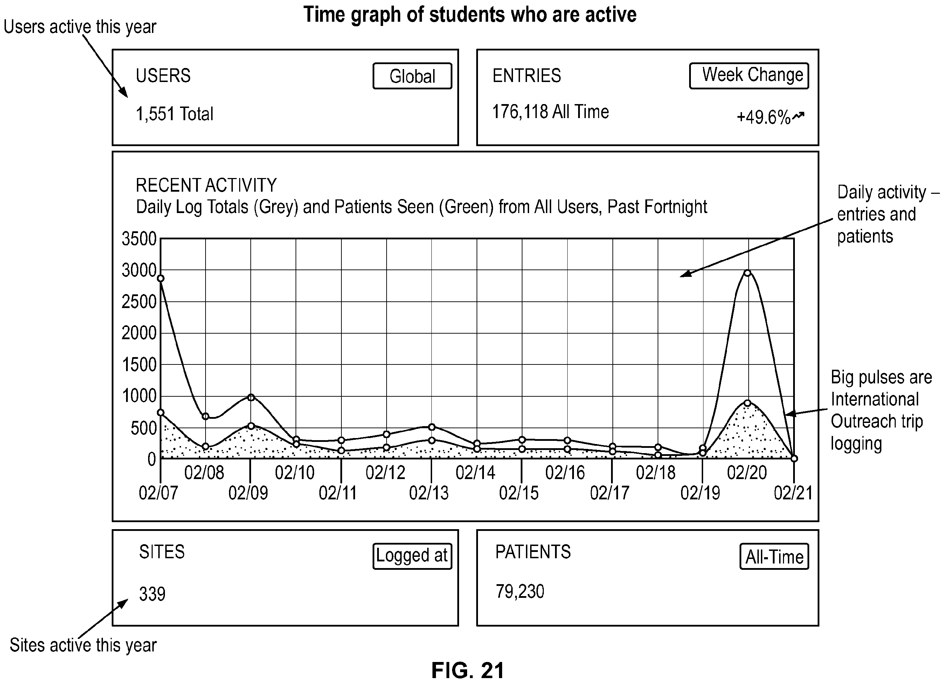

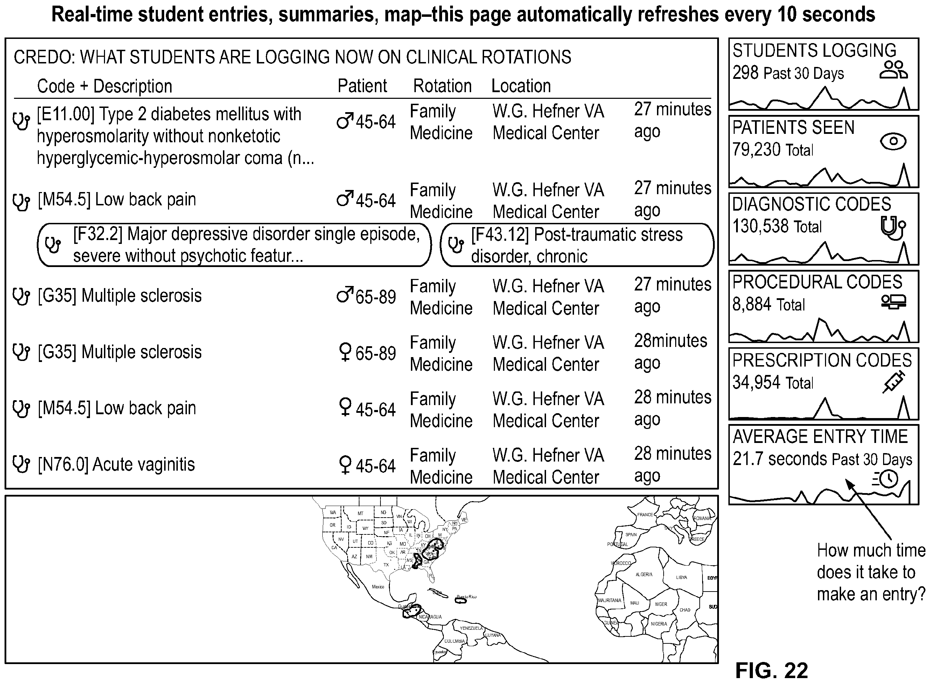

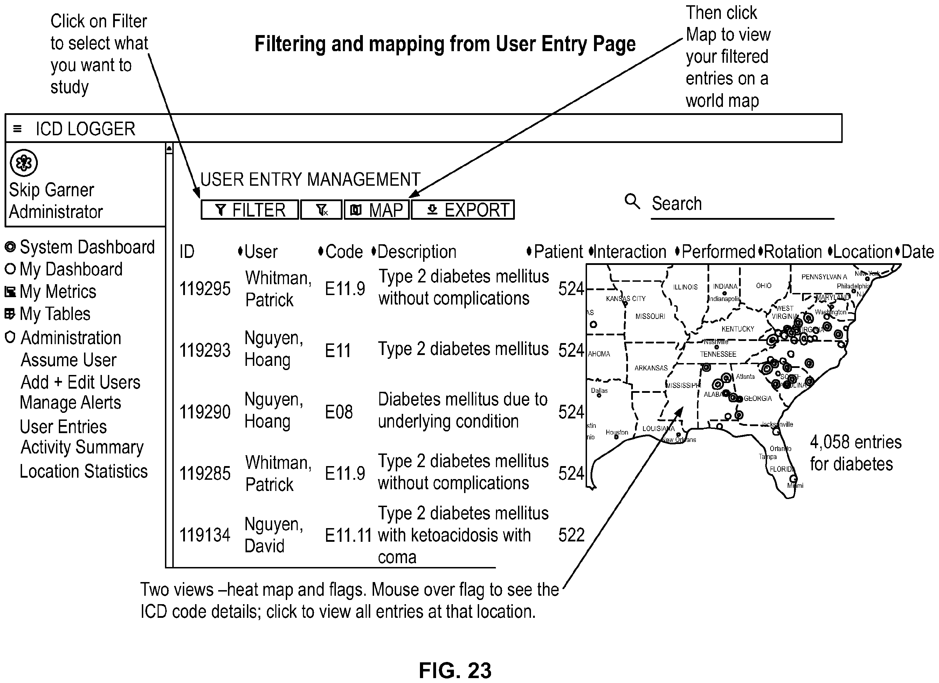

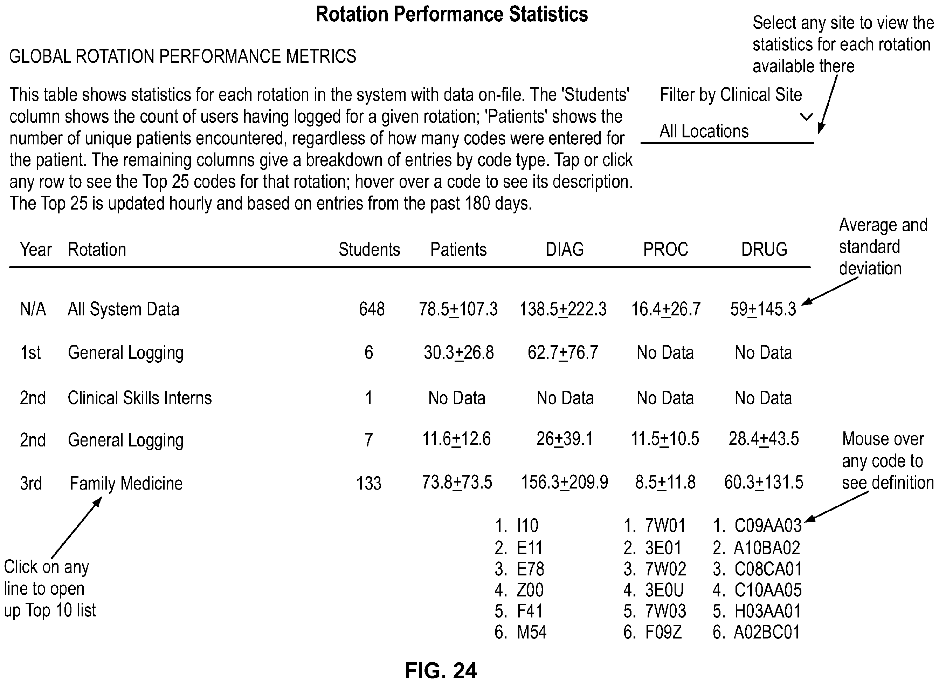

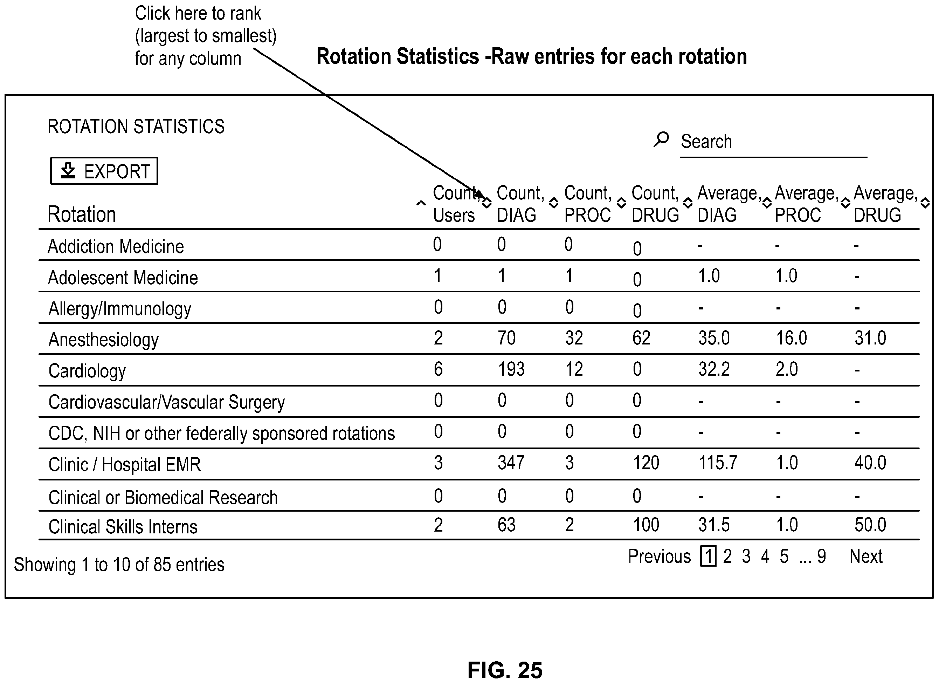

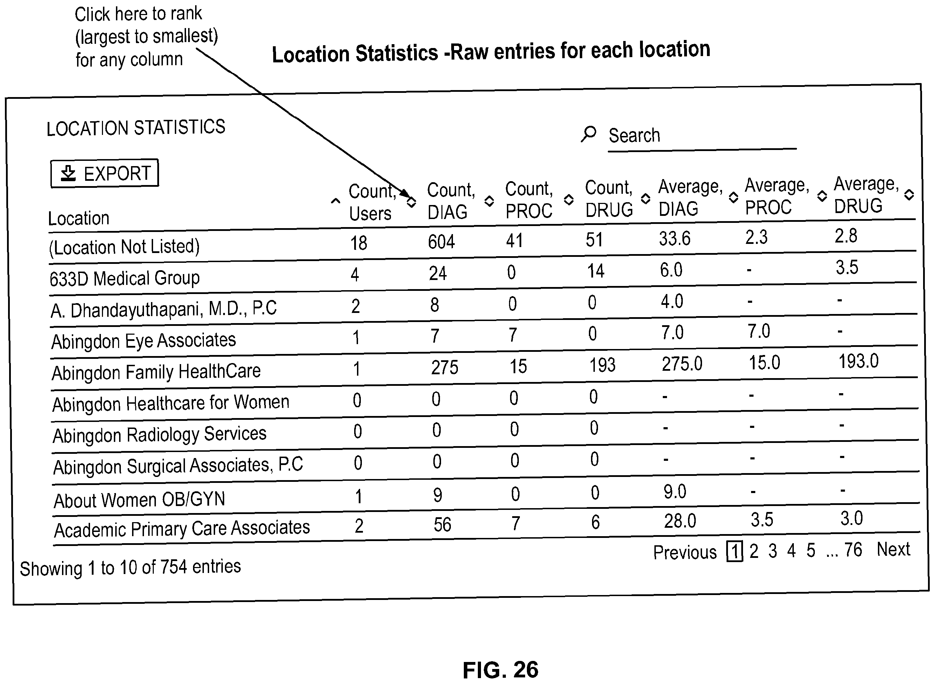

Figure 21 shows an exemplary screen illustrating a time graph of active students. Figure 22 shows an exemplary screen illustrating real-time student entries, summaries, map which may refresh every 10 seconds. Figure 23 shows an exemplary screen illustrating filtering and mapping from a User Entry Page. Figure 24 shows an exemplary screen illustrating rotation performance statistics. Figure 25 shows an exemplary screen illustrating rotation statistics-raw entries for each location. Figure 26 shows an exemplary screen illustrating location statistics-raw entries for each location.

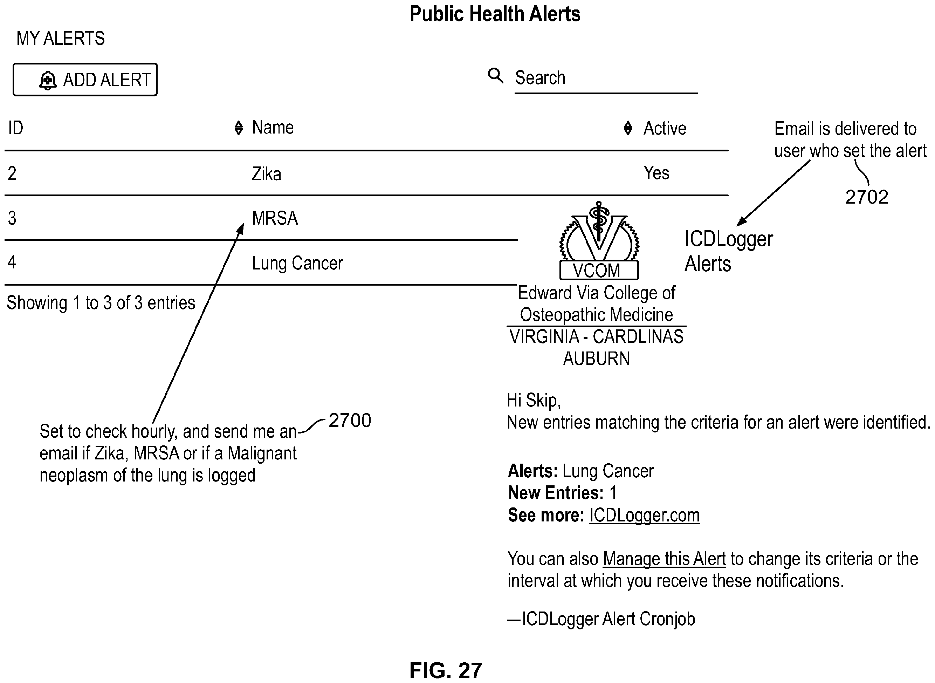

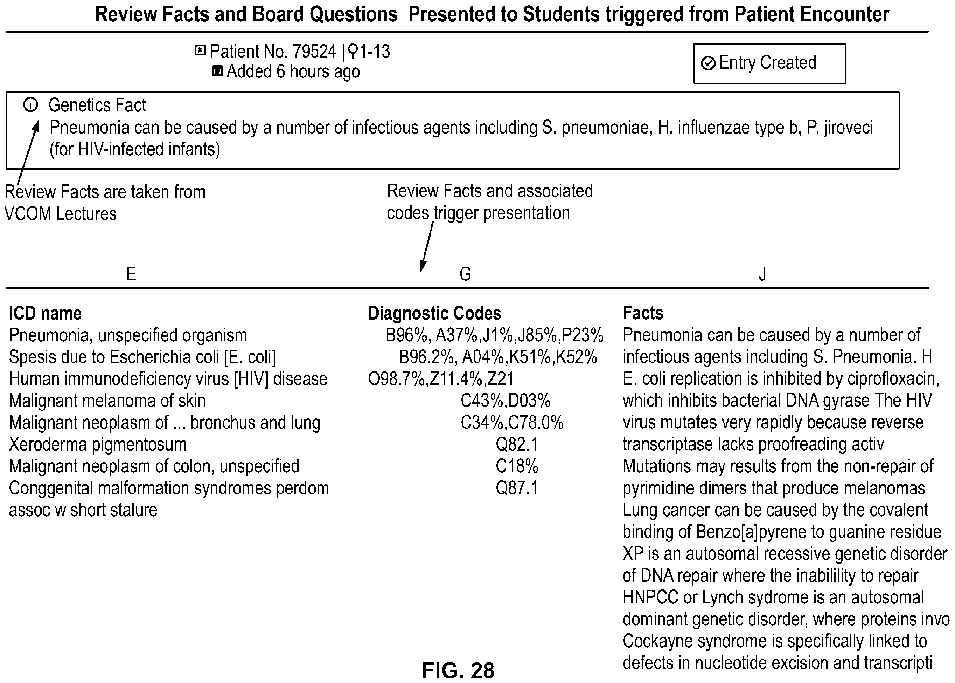

Figure 27 shows an exemplary screen illustrating the utility of the present invention for issuing public health alerts. As shown, the system may be programmed to check hourly or for some other predetermined period for a known health hazard 2700. An email or some other form of an alert such as a text message may be sent to a predetermined user or users 2702. Figure 28 shows an exemplary screen illustrating review facts and board questions presented to students triggered from a patient encounter.

### Authoring software tool, CASEAGE, and integrated dynamic interactive PRESENTER for Case-based Learning

in yet other embodiments, the present invention provides a curriculum management system where medical school cases can be created, edited, reviewed/verified, and then made available for different uses. Cases include all standard components, including case description, metadata, case presentation through a management plan. The system enables the incorporation of multimedia, including sound, video, and other data types in the presentation of a case. Each case includes questions, annotated answers, and references. These questions are used to make the presentation of the case dynamic, as correct answers, in addition to being used for grading, and can be used to release other case components as the case unfolds when being completed by students. Other questions are used as reading comprehension checks as the case progresses. It has been designed to track the case author's progress in developing and validating a case as well as facilitates team feedback and interaction. Lastly, cases can be presented in CREDO, and dynamically linked to patient encounter logging or used as part of curriculum presentation.

This embodiment of the present invention serves as a platform within the web-based CREDO system for the delivery of case-based learning. This learning format allows for both horizontal and vertical integration of information. Case-based learning is supported by the principles of adult education as it provides self-directed cases, occur in an interactive environment, provide immediate feedback, and concrete application of knowledge through board style questions to increase learner engagement and retention of information. The incorporation of case-based, board-style questions and non-cased-based, reading comprehension checks follows Bloom's taxonomy which is a hierarchical model of knowledge acquisition. Case-based, -board-style questions require a higher level of cognitive processing which facilitates knowledge consolidation, retention, and improvement on question performance.

In yet other embodiments, the present invention provides a curriculum management system where medical cases can be created, edited, reviewed/verified, and then made available for different applications. Cases include case description, metadata, and case presentation through assessment and management plan. This embodiment enables the incorporation of multimedia, including sound, video, and other data types in the presentation of a case. Each case includes questions, annotated answers, and references. These questions are used to make the presentation of the case dynamic, as correct answers, in addition to being used for grading, functions to release other case components as the case unfolds that emulate direct interaction and stimulate critical thinking.

In other aspects, the embodiment is adapted to track the case author's progress of developing and validating a case and facilitates team feedback and interaction. Developers can have various assigned roles, including the primary author(s), validator(s), and administration. The primary author team is responsible for building the case content, developing questions, and dynamic question control logic. The validator(s) have responsibility for checking the content as well as the presentation of the case for accuracy and usability, and final validation sign off before deployment. Administrators can assign roles, create/initiate cases on which authors and validators work, and have final authority to deploy a case as complete and validated. Administrators can also request updates and track update progress.

This embodiment of the present invention was built around a modern browser-based interface that has flexibility and extensibility. The web-browser page has various tabs (Case Description, Learning Objectives, Learning Materials, and in the Case Build) where authors can enter questions that users must answer. Questions are multiple-choice with up to five possible answers following the format for medical board exam-style questions, with answers, including one that is marked as the correct answer, along with rationale for each correct and incorrect answer. Questions and answers can have multimedia associated with them. Also, question logic controls allow for reading check questions to be integrated within the case to ensure learner engagement.

The presentation of the case is made dynamic and autonomous using questions and corresponding the corresponding continuation policy. The Continuation Policy choices include question optional (answer does not affect revealing next parts of the case), attempt required (answer must be attempted, but not require to be correct to reveal the next parts of the case), and correct response required (a correct answer is required to reveal the next parts of a case). Controlling case presentations in this way creates a dynamic environment, as opposed to providing a case in its entirety such as a case publication. This approach also forces the student user to answer questions at appropriate times to ensure mastery of the material before moving forward and provides a feedback mechanism that can require additional review of material if mastery is not achieved.

The authoring tool breaks the case building into four major sections:
Case Description - Here the case is given a name, and associate it with appropriate ICD-10 codes, courses, and initially control the status of your case (in development....to deployed).
Learning Objectives - In this section, the major learning goals for a case are entered. Authors can also assign a Category to the Learning Objective, such as Diagnostic Evaluation, Pathology, etc.
Learning Materials - Authors can assemble materials that they require users to review before they begin to progress through the case. Authors can provide assigned readings, link to online resources, attach and upload specific documents, and multimedia.
Case Build - It is under this section that contains the bulk of a case. There are numerous sections, from Case Presentation to Management Plan to Learning/educational materials for the caretaker and patient. There you can also enter references used in the preparation of the case.

Once a case is developed, it must be deployed for use by learners. With the case and its contents in a uniform format contained in a database, it can be made available in different ways, including a simple export of the content of the database for import in any variety of presentation tools. It is also possible to dynamically link downstream applications that present the case to the user directly from the case build database, thus assuring that the latest version of a case is always given to learners. In a preferred embodiment, the present invention includes one such tool, called PRESENTER, inside CREDO system described above. The goals and utility of the presentation tool are to:
1. Quantify the learner's progress, competency, and proficiency.
2. Understand common trends among students to address learning weaknesses.
3. Use this information to assess student progress, provide feedback on performance and material mastery to the students.
4. Capture and monitor progress in real-time
5. Present to the user in a consistent way a collection of well-organized/formatted and indexed medical school cases

The PRESENTER provides the user with real-time feedback about their understanding and mastery of the material using knowledge checks in the form of questions created by the case creator. This can be as simple as a reading comprehension assessment or higher-order questions geared at gauging a user's readiness for final course testing. It allows the creator to provide feedback for each of the multiple-choice answers as to why each was correct or incorrect. With each knowledge check, the creator can choose to allow the user to progress or try the question again until the desired answer is correctly selected. This allows a user the chance to go back and review the material again if they did not fully comprehend a section. Questions can be tallied at the end to provide the user with the overall information regarding their performance so that they have objective information about their understanding of the material to allow self-assessment if further study is needed.

The PRESENTER can track user data by collecting the information from inserted knowledge checks. This can allow the creator to compile this data and allow them to adjust the case presentation to address trends in student weaknesses. This trending could also be used by a creator to adjust their course curriculum if there appear to be areas where students are consistently struggling. This provides a valuable tool for a course instructor to address deficiencies in understanding before final course testing. It can also help instructors to re-evaluate course structure at a high resolution based on student understanding of higher-yield material versus complementary and supporting information.

Very often there is little feedback in course understanding and mastery before a final course examination. With students engaged in multiple courses, they often are taught by multiple instructors with varying teaching styles and priorities for what is considered high-yield and testable material. By providing short case-based learning modules with included knowledge checks, the student can begin to understand the style and priority of the instructor in addition to higher yield topics for board exam questions. This provides another avenue to present course objectives and material that can be used to assist a wider variety of student learning styles.

The PRESENTER has the flexibility in how it offers available "deployed" cases, as either a list from which students can select cases to study, or it can wisely offer appropriate cases. PRESENTER contains the capability to automatically trigger the offering of an appropriate case to couple, for example, the logging of patient encounter diagnoses and procedures to the presentation of cases to re-enforce background knowledge associated with the immediate trainee-patient interaction and hands-on learning. This provides the user with the ability to match real-time patient encounters and interactions with additional information about a case. It could also provide information about different patient presentations that the user did not personally encounter to help to build and reinforce the real-time active learning of the student. This can be a very powerful tool and resource for the student to quickly compile information about a case, disease process, patient presentations, and treatment options. This information and the cases which can be compiled from creators from different disciplines allowing for a wider range of information and knowledge at the user's fingertips. This could also provide a great resource for students to quickly reference how similar cases might be managed across different disciplines aiding the student not only in their understanding of a disease process but the interactions and consultations from different specialties. Knowing how different specialties interact, consult, and manage patients is often overlooked in formal training and is often learned by a student through experience. This tool could provide a significant advantage to a student new to the clinical setting so that they can better integrate into the medical care team.

In a preferred embodiment, case authors are required to annotate each case with appropriate ICD-10 codes. In turn, these ICD-10 codes are the link that allows the automatically triggered offering of a case to students logging their recent clinical patient encounter activities, or as one way to search a list of available deployed cases.

### Continuous, Sustainable Worldwide Scalable Surveillance for Early Detection of Emerging Infectious Diseases and Monitoring Response

In yet other embodiments, the present invention may be used to predict and recognize contagious events such as COVID-19. In a preferred embodiment, the system runs on any browser-based hardware from a PC to a smartphone. Data collection is centralized on a HIPAA compliant cloud with a variety of spatial and temporal readouts, graphics and reports.

In its various embodiments, the present invention allows a user to predict the next pandemic which may be prevented if a new and emerging highly infectious disease/pathogen is detected early and intercepted. While it is unknown where the next infectious disease will emerge, it is likely to come from a remote location where people live close to animals. The first signs of such will appear when those infected show up at their local clinic, which in most of the 2nd and 3rd world are not sophisticated, lacking connectivity of medical records and in countries where the reporting structure is spotty at best. By providing a system that is easy to deploy and use, embodiments of the present invention can capture real-time data, while clinicians throughout the world will obtain a desired, but currently out of reach capacity - electronic record-keeping/backup and communication with a larger medical community.

In a preferred embodiment, the present invention provides a solution based on the embodiments described above. Moreover, this particular embodiment of the present invention is also based around WHO ICD codes to provide a common universal medical vocabulary which can capture diagnoses, procedures and drugs during patient encounters, and then use those codes as an index to associate a variety of information (learning objectives, basic science facts, educational materials, public health alerts) to which we have assigned appropriate ICD codes. This allows the embodiment to dynamically link entries to objectives to provide a more informed real-time clinical performance assessment and deliver appropriate board-level review cases, multimedia and challenge questions with rationale and references while tracking review performance. As a public health surveillance tool, these universal codes enable compatibility with many existing clinical and surveillance systems, while enabling the connection to relevant information and easing the creation of multi-lingual versions. The system of the present embodiment includes a variety of real-time reports, data filtering and downloading, including geospatial mapping of entries, temporal trending, and system email alerts upon detection of specified ICD-10 codes or frequency trends in codes.

### Alerts Generated by CREDO Patient Encounter Data

To function as a syndromic surveillance system, the present invention monitors diagnostic codes logged by medical professionals and various locations in real-time for each patient encounter. An algorithm may then be applied to the syndromic data to detect unusual levels of disease indicating a potential outbreak. The data may also be compared to prior years. For this example, COVID-19 was compared to last year's flu season, and during this time the respiratory and flu-like illness diagnosis codes logged was more than double last year's rate. Thus, the analysis algorithm is designed to trigger alerts when spikes exceed normal rates.

In other embodiments, the present invention includes time-dependent graphical monitors that can be set to review patient encounter data for any ICD-10 diagnoses. For more customized analyses, all data can be exported in Excel or other formats so users can input into their analysis environment, including Excel, R, and other statistical packages.

To supplement the standard surveillance capabilities of the present invention, an additional surveillance mode is based on self-reporting by users of their infectious disease status, and the status of their contacts. Each report captures the time and geospatial location where the entry was made and relevant health and contact data.

In other aspects, the present invention may provide one or more dashboard summaries that provide the status of all users (including the ability to export in its entirety). The dashboard may further include a "risk index" for users and administrators; e.g. those that had direct exposure would be considered as high risk. This index can be used as a trigger to drive further actions, including, for example, an alert that is automatically sent to a healthcare professional.

In other embodiments, the present invention includes systems and methods wherein all operations are real-time; data are collected over a very large number of distributed rural and urban sites and thus cover a large region of the United States/World without the need to extract and merge data from disparate hospital Electronic Medical Records systems; patient encounter data is uniformly collected by clinically trained professionals; data entry is very easy and very fast (∼12 seconds per entry); and self-reporting health status and contact tracing is very simple and fast, suitable for lay users.

### ICD logging system to supplement an EMR

The CREDO system described above may also be used to implement an electronic medical record (EMR) system that has the potential to advance medical technology in care to remote geographic locations where the Internet and other resources are scarce or unavailable. In this embodiment, there is the Logger page, where users can log patient diagnoses, procedures, and prescriptions, and then the Dashboard, where users can view and search through the entries that they have made as well as generate appropriate reports.

In a preferred embodiment, the present invention provides a welcome screen leading to pages where a user can access their account or log out, if necessary. On another page, there are links to the support team, user instructions, FAQ documents and a brief "about" page. On the logging page, the user has the option of allowing geolocation to be activated, to provide better data collection for the development team's use. From there, the user can navigate between the Diagnosis, Procedures, and Drug pages. On the Diagnosis page, users can enter ICD 10-CM codes by selecting categories, typing in a diagnosis or synonyms, searching the top 10 diagnoses, or choosing the most recent diagnosis entries. Users also log whether the patient is new or is an existing one, the gender and age, and finally enter any notes that do not disclose confidential information. In the Procedures page, like the Diagnosis page, users can enter the ICD-10-PCS code through the four different methods and enter the demographic information. They can also include the engagement level, either "Observed" or "Performed", which is primarily for the medical students. On the Drug page, users can enter the WHO-ATC code through the four methods and enter the demographic information. Dosages can be placed in the notes section. On the Dashboard page, users can see how many other users are using the Logger, their statistics of logging and then all the entries they have made. If necessary, they may edit information by clicking on an entry. Users can also export information into spreadsheets, charts and tables, filtered to their needs to aid in creating reports. A locally made spreadsheet that incorporates patient identifiers obtained from the local EMR system, HealthMaster program, was created to associate personal contact information to the CREDO ICD Logger Internal Patient Number (identifier), thereby linking the software and the web-based program. The spreadsheet is encrypted, to prevent confidential information from being exposed, and only authorized personnel may access it. The spreadsheet allows clinicians to make new or review ICD entries or information for a specific patient via the CREDO ICD Logger Internal Patient Number. The Logger provides anonymous patient-specific data and is a valuable backup storage method. It also allows for open comparison to other sites in which students are logging their clinical experiences. Together, the two databases (the CREDO ICD Logger and the local spreadsheet) provide secure and reliable access to patients' information.

While the foregoing written description enables one of ordinary skill to make and use what is considered presently to be the best mode thereof, those of ordinary skill will understand and appreciate the existence of variations, combinations, and equivalents of the specific embodiment, method, and examples herein. The disclosure should therefore not be limited by the above-described embodiments, methods, and examples, but by all embodiments and methods within the scope and spirit of the disclosure.

The present teaching may also extend to provide a system implemented on a distributed computer network for implementing an electronic medical record (EMR) system, the system comprising:
a plurality of electronic input devices, each of said input devices having an interface for interacting with a user including an input screen having data input fields, selection fields and activation buttons and output screens;
one or more back-end databases;
said input devices in communication with said one or more back-end databases, said database configured to store input data from said input devices and to provide output data to said input devices;
ICD codes associated patient diagnoses, procedures and prescriptions; and
said ICD codes included in said input data provided to said one or more back-end databases.

## Claims

1. A system implemented on a distributed computer network for surveillance and early detection of emerging infectious diseases and monitoring responses, the system comprising:
a plurality of electronic input devices, each of said input devices having an interface for interacting with a user including an input screen having data input fields, selection fields and activation buttons and output screens;
one or more back-end databases;
said input devices in communication with said one or more back-end databases, said database configured to store input data from said input devices and to provide output data to said input devices;
ICD codes associated with a disease and used to identify and capture patient encounters by healthcare providers, said encounters included in said input data provided to said one or more back-end databases; and
an algorithm may then be applied to the collected data to detect unusual levels of a disease indicating a potential outbreak.

2. The system of claim 1 further including time-dependent graphical monitors that can be set to review patient encounter data for any ICD-10 diagnoses.

3. The system of claim 1 further including a self-reporting mode allowing users to input their infectious disease status, and the status of their contacts.

4. The system of claim 3 wherein each report captures the time and geospatial location where the entry was made and relevant health and contact data.

5. The system of any preceding claim wherein said disease is COVID-19.

6. The system of any preceding claim further configured to permit healthcare trainees to search ICD codes using at least one of hierarchical menus, using keywords, or by presenting frequently entered ICD codes.

7. The system of any preceding claim further configured to permit healthcare trainees to link data entries to a selected ICD code.

8. The system of any preceding claim further configured to permit healthcare trainees, for each encountered patient, the ability to input: 1) the ICD code that describes their diagnosis and treatment procedure; 2) codes for WHO listed and/or FDA approved drugs; 3) the patient gender and age; 4) free-text notes; 5) the clinic/hospital and rotation type as entered by the user and whether the training environment is a hospital or ambulatory; 6) their precise location; and 7) date/time of entry.

9. The system of any preceding claim further configured to include a personally identifiable information detection system, said detection system prevents entry of personally identifiable information.

10. The system of any preceding claim wherein said back-end database is configured to generate reports that enable the tracking and editing of data entries.

11. The system of any preceding claim further configured to allow administrators to monitor the cumulative entries from all trainees, or to monitor trends and trigger alerts from defined thresholds, or to include displays adapted to monitor current activities being logged and to provide statistics that track and graph system-wide usage.

12. A system implemented on a distributed computer network for capturing experiences of healthcare trainees, the system comprising:
a plurality of electronic input devices, each of said input devices having an interface for interacting with a user including an input screen having data input fields, selection fields and activation buttons and output screens;
one or more back-end databases;
said input devices in communication with said one or more back-end databases, said database configured to store input data from said input devices and to provide output data to said input devices; a login system including user identification routines to establish user identity and user system access status;
a common language to identify and capture clinical or training encounters by healthcare trainees, said encounters included in said input data provided to said one or more back-end databases, said common language is ICD codes; and
predetermined learning objectives, medical information or medical board review questions that are presented to a healthcare trainee.

13. The system of claim 12 wherein said predetermined learning objectives are medical case.

14. The system of claim 12 further configured to provide real-time interactive information to a healthcare trainee associated with an inputted ICD code.

15. The system of claim 13 further configured to include a collection of databases containing annotated ICD codes to connect patient encounters, learning materials, review materials, and performance metrics.
